(19) Europäisches Patentamt
European Patent Office
Office européen des brevets

(11) EP 4 684 778 A1

# (12) EUROPEAN PATENT APPLICATION

published in accordance with Art. 153(4) EPC

(43) Date of publication:
**28.01.2026 Bulletin 2026/05**

(21) Application number: **24799868.5**

(22) Date of filing: **23.04.2024**

(51) International Patent Classification (IPC):
***A61K 9/28*** (2006.01) ***A61K 31/403*** (2006.01)
***A61K 31/4015*** (2006.01) ***A61K 31/4025*** (2006.01)
***A61P 31/14*** (2006.01)

(86) International application number:
**PCT/CN2024/089353**

(87) International publication number:
**WO 2024/227410 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.05.2023 CN 202310494598**
**16.05.2023 CN 202310555855**
**13.06.2023 CN 202310699201**
**13.06.2023 CN 202310703297**

(71) Applicant: **Guangdong Raynovent Biotech Co., Ltd.**
**Huangpu District**
**Guangzhou 510700 (CN)**

(72) Inventors:
- **CHEN, Xiaoxin**
  **Guangzhou, Guangdong 510700 (CN)**
- **LIU, Zhuowei**
  **Guangzhou, Guangdong 510700 (CN)**
- **WAN, Chunxi**
  **Guangzhou, Guangdong 510700 (CN)**
- **YANG, You**
  **Guangzhou, Guangdong 510700 (CN)**
- **XU, Xin**
  **Guangzhou, Guangdong 510700 (CN)**
- **OU, Kunyong**
  **Guangzhou, Guangdong 510700 (CN)**

(74) Representative: **Clark, Claudia et al**
**Redl Life Science Patents**
**Donau-City-Straße 11**
**1220 Wien (AT)**

## (54) PHARMACEUTICAL COMPOSITION AGAINST SARS-COV-2 AND PREPARATION METHOD THEREFOR

(57) Provided is a pharmaceutical composition, comprising a compound of formula I or a pharmaceutically acceptable salt thereof and an auxiliary material. The auxiliary material comprises one or more of a filler, a binder, a glidant, a disintegrant and a lubricant. The pharmaceutical composition has the feasibility of preparation process, excellent properties of preparations, good stability and good dissolution, and meets the pharmaceutical standards.

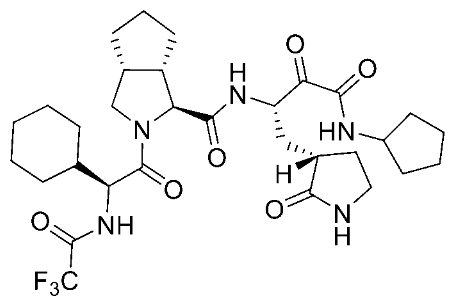

I

EP 4 684 778 A1

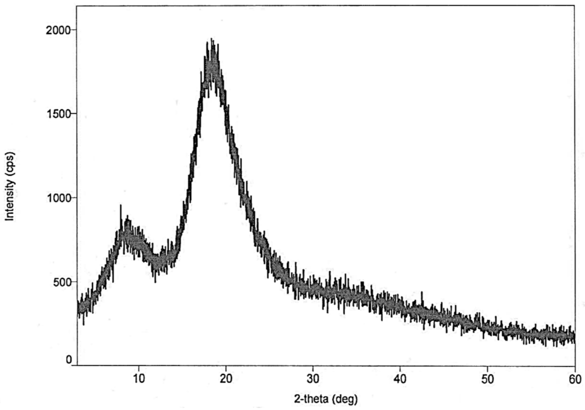


FIG. 1

## Description

### TECHNICAL FIELD

The present disclosure relates to the field of pharmaceutical preparations, in particular to a pharmaceutical composition against SARS-CoV-2 and a preparation method therefor.

### BACKGROUND

Coronaviruses are a family of single-stranded positive-sense RNA viruses with viral envelopes, belonging to the order Nidovirales. The coronavirus family contains pathogens of many animal species, including humans, horses, cattle, pigs, birds, cats, and monkeys, and has been known for more than sixty years. For example, the isolation of the prototype strain of murine coronavirus, JHM, was reported in 1949. Coronaviruses are common viruses that commonly cause mild to moderate upper respiratory illnesses in humans and are named for the crown-shaped spikes on the surface of their envelope. The SARS pandemic of 2003 led to the emergence of SARS-CoV-1, a pathogen that causes severe respiratory infections. The subsequent surge in coronavirus research led to the discovery of two other human coronaviruses with mild pathogenicity, HCoV-NL63 and HCoV-HKU1. MERS-CoV, which emerged in 2012, is a member of this group of pathogens that causes severe respiratory infections. Although SARS-CoV-1 and MERS-CoV are highly lethal pathogens, the public health, social, and economic losses they have caused are negligible compared to those caused by SARS-CoV-2. SARS-CoV-2 is a newly emerged human CoV pathogen that causes COVID -19. It has caused the COVID-19 pandemic, which is second only to the 1918 influenza pandemic and has had a catastrophic effect worldwide. As of April 2023, the total number of COVID-19 cases worldwide has exceeded 680 million, with more than 6.85 million deaths. In order to mitigate the catastrophic damage of COVID-19 to public health, society, and the economy, it is crucial to find timely treatment options.

Coronaviruses are enveloped, positive-sense, single-stranded RNA viruses. The genomic RNA of CoV has a 5'-cap structure and a 3'-poly-A tail, and contains at least six open reading frames (ORFs). The first ORF (ORF la/b) directly translates two polyproteins: ppla and pplab. These polyproteins are processed into 16 nonstructural proteins by the 3C-like protease (3CLpro), also known as the major protease (Mpro). These nonstructural proteins are involved in the production of subgenomic RNAs encoding four structural proteins, namely, envelope proteins, membrane proteins, spike proteins, and nucleocapsid proteins, as well as other accessory proteins. Therefore, 3C-like proteases are known to play a key role in the coronavirus life cycle.

3C-like protease is a cysteine protease involved in most cleavages within the precursor polyprotein. The active 3C-like protease is a homodimer containing two protomers with a Cys -His dimer located between domains I and II. The 3C-like protease is conserved among coronaviruses, and the substrates of 3C-like protease share several common features among different coronaviruses. Since 3C-like protease has no human homologs, it is an ideal antiviral target.

Paxlovid, an oral anti-SARS-CoV-2 drug developed by Pfizer, Inc., is composed of 300 mg (2 tablets of 150 mg) Nirmatrelvir and 1 tablet of 100 mg Ritonavir. The insert of the listed drug Paxlovid discloses that Nirmatrelvir tablets consist of Nirmatrelvir, microcrystalline cellulose, lactose monohydrate, cross-linked sodium carboxymethyl cellulose, colloidal silica, sodium stearyl fumarate, and hydroxypropyl methylcellulose.

Leritrelvir tablet (Trade name: Le Rui Ling), an oral anti-SARS-CoV-2 drug developed by Guangdong Raynovent Biotech Co., Ltd., is a 3C-like protease inhibitor that does not require combined use with Ritonavir and is currently available in China.

Chinese Patent ZL202211095326.8 discloses a series of keto-amide derivatives, such as the compound of Formula I, which have good activity against SARS-CoV-2 Mpro protease.

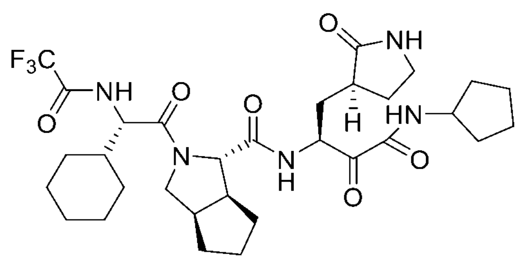

Formula I

Although Chinese Patent ZL202211095326.8 discloses *in vitro* tests of this series of keto-amide compounds, proving that they have strong anti-SARS-CoV-2 effects and good pharmacokinetics, there are currently only relevant activity studies on the compounds and pharmaceutically acceptable salts thereof, and there have been no reports on

pharmaceutical compositions or pharmaceutical preparations with the above compounds as active ingredients. Therefore, further research and application of the above compounds are imminent.

## SUMMARY OF THE INVENTION

The technical problem to be solved by the present disclosure is to overcome the deficiencies in the prior art and provide a pharmaceutical composition against SARS-CoV-2 and a preparation method therefor.

To achieve the above-mentioned purpose, the present disclosure has conducted a large number of experimental studies and improvements on the existing formulations and processes of preparations, thereby proposing a pharmaceutical composition of the present disclosure comprising a compound of Formula I or a pharmaceutically acceptable salt thereof, which has good feasibility of preparation process and high stability, can be orally administered to treat COVID-19, and its preparation method is simple to operate and suitable for industrial production.

The specific technical solution of the present disclosure is as follows: a pharmaceutical composition including a compound of Formula I or a pharmaceutically acceptable salt thereof and an auxiliary material(an excipient), the auxiliary material includes one or more of a filler, a binder, a glidant, a disintegrant and a lubricant, the filler is one or more of lactose, anhydrous lactose, microcrystalline cellulose, mannitol or starch, the binder is one or more of hydroxypropyl methylcellulose, hydroxypropyl cellulose, Povidone, hydroxyethyl cellulose, sodium carboxymethyl cellulose or polyvinyl alcohol, the glidant is one or more of colloidal silica, talc or micronized silica gel, the disintegrant is one or more of low-substituted hydroxypropyl cellulose, cross-linked sodium carboxymethyl cellulose, sodium carboxymethyl starch or cross-linked Povidone, the lubricant is one or more of magnesium stearate, calcium stearate or sodium stearyl fumarate, a mass ratio of the compound of Formula I to the filler is 1:1.0-2.5, a mass ratio of the compound of Formula I to the binder is 1:0.01-0.05, a mass ratio of the compound of Formula I to the glidant is 1:0.01-0.03, a mass ratio of the compound of Formula I to the disintegrant is 1:0.10-0.30, and a mass ratio of the compound of Formula I to the lubricant is 1:0.01-0.10.

Unless otherwise specified, throughout the context of the present disclosure, the mass of the compound of Formula I refers to the mass of the compound of Formula I itself or the mass of the compound of Formula I in the pharmaceutical composition of the compound of Formula I.

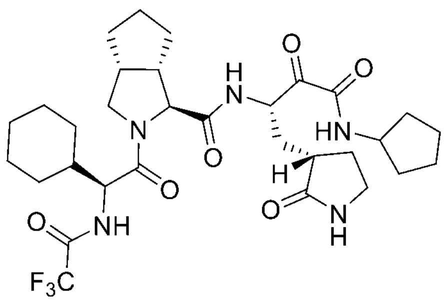

Formula I

Alternatively, the present disclosure provides a pharmaceutical composition including a compound of Formula I or a pharmaceutically acceptable salt thereof and an auxiliary material, the auxiliary material includes one or more of a filler, a binder, a glidant, a disintegrant and a lubricant; the filler is one or more of lactose, anhydrous lactose, microcrystalline cellulose, mannitol or starch, the binder is one or more of hydroxypropyl methylcellulose, hydroxypropyl cellulose, Povidone, hydroxyethyl cellulose, sodium carboxymethyl cellulose or polyvinyl alcohol, the glidant is one or more of colloidal silica, talc or micronized silica gel, the disintegrant is one or more of low-substituted hydroxypropyl cellulose, cross-linked sodium carboxymethyl cellulose, sodium carboxymethyl starch or cross-linked Povidone, the lubricant is one or more of magnesium stearate, calcium stearate or sodium stearyl fumarate, a mass ratio of the compound of Formula I to the filler is 1:1.0-2.5, a mass ratio of the compound of Formula I to the binder is 1:0.01-0.05, a mass ratio of the compound of Formula I to the glidant is 1:0.01-0.03, a mass ratio of the compound of Formula I to the disintegrant is 1:0.10-0.30, a mass ratio of the compound of Formula I to the lubricant is 1:0.01-0.10, and the pharmaceutical composition has a moisture content not exceeding 5.0%.

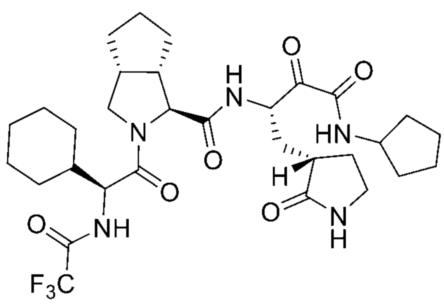

Formula I

Alternatively, the present disclosure provides a pharmaceutical composition including a compound of Formula I or a pharmaceutically acceptable salt thereof and an auxiliary material, the auxiliary material includes one or more of a filler, a binder, a glidant, a disintegrant and a lubricant, the filler is one or more of lactose, anhydrous lactose, microcrystalline cellulose, mannitol or starch, the binder is one or more of hydroxypropyl methylcellulose, hydroxypropyl cellulose, Povidone, hydroxyethyl cellulose, sodium carboxymethyl cellulose or polyvinyl alcohol, the glidant is one or more of colloidal silica, talc or micronized silica gel, the disintegrant is one or more of low-substituted hydroxypropyl cellulose, cross-linked sodium carboxymethyl cellulose, sodium carboxymethyl starch or cross-linked Povidone, the lubricant is one or more of magnesium stearate, calcium stearate or sodium stearyl fumarate, and the pharmaceutical composition exhibits a dissolution rate of 70.0% or more at 60 min in a phosphate buffer solution with a pH of 6.8 as a dissolution medium.

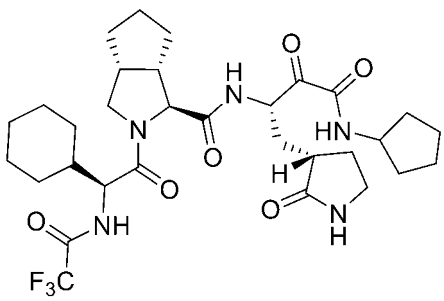

I

The present disclosure provides a pharmaceutical composition against SARS-CoV-2 and a preparation method therefor. The effect is achieved by combining the physicochemical properties of the active ingredient and the compatibility test results of the auxiliary materials to select appropriate auxiliary materials for the preparations. The type and/or amount of the formulation have significant impact on the preparation process and the quality of the preparations. The auxiliary materials used in the present disclosure include a filler, a wetting agent, a binder, a disintegrant, a lubricant, and the like. According to the present disclosure, a filler is introduced into the pharmaceutical composition containing the compound of Formula I, which includes one or more of lactose, anhydrous lactose, microcrystalline cellulose, mannitol or starch, and preferably is a mixture of lactose and microcrystalline cellulose, which can ensure the oral preparations of the compound of Formula I to achieve the quality or volume standard for preparing a solid dosage form. According to the present disclosure, a binder is introduced into the pharmaceutical composition containing the compound of Formula I, which includes one or more of hydroxypropyl methylcellulose, hydroxypropyl cellulose, Povidone, hydroxyethyl cellulose, sodium carboxymethyl cellulose or polyvinyl alcohol, preferably hydroxypropyl methylcellulose; the binder can bind the active ingredient with the auxiliary materials, and when the binder is hydroxypropyl methylcellulose, due to the surface activity of hydroxypropyl methylcellulose, it helps to enhance the dissolution of the drug. According to the present disclosure, a glidant is introduced into the pharmaceutical composition containing the compound of Formula I, which includes one or more of colloidal silica, talc or micronized silica gel, preferably colloidal silica; the glidant can reduce sticking, and lower friction between granules and between tablets and die wall, thus improving the fluidity of granules and making the tablet surface smooth and beautiful. According to the present disclosure, a disintegrant is introduced into the pharmaceutical composition containing the compound of Formula I, which includes one or more of low-substituted hydroxypropyl cellulose, cross-linked sodium carboxymethyl cellulose, sodium carboxymethyl starch or cross-linked Povidone, preferably cross-linked sodium carboxymethyl cellulose; the disintegrant can rapidly break the tablet into fine particles in the gastrointestinal fluid, thereby enabling the functional components to be dissolved and absorbed quickly to exert their

effects; when the disintegrant is cross-linked sodium carboxymethyl cellulose, the brittleness of the tablet is not affected regardless of whether the disintegrant is added intra-granularly or extra-granularly. According to the present disclosure, a lubricant is introduced into the pharmaceutical composition containing the compound of Formula I, which includes one or more of magnesium stearate, calcium stearate or sodium stearyl fumarate, preferably magnesium stearate; the lubricant can evenly distribute the pressure during tableting and make the density of the tablet uniform.

The present disclosure also provides a pharmaceutical composition against SARS-CoV-2 and a preparation method therefor, the effects of which can be achieved by controlling the amount of the auxiliary materials for the preparations. Specifically, an increase in the amount of the auxiliary materials is beneficial for enhancing the compressibility and reducing the possibility of sticking. However, the increase in the amount of the auxiliary materials will lead to high tablet weight, which is not favorable for administration to patients and has additional negative effects on the performance of the preparations, for example, too much disintegrant may cause excessive lubrication of the tablet and delay dissolution. Unless otherwise specified, the amount of active ingredients in the preparation formulation of the present disclosure is calculated based on the compound of Formula I (on a dry and pure basis). When a corresponding auxiliary material is selected, the amount of the auxiliary material is: the mass ratio of the compound of Formula I to the filler is 1:1.0-2.5, the mass ratio of the compound of Formula I to the binder is 1:0.01-0.05, the mass ratio of the compound of Formula I to the glidant is 1:0.01-0.03, the mass ratio of the compound of Formula I to the disintegrant is 1:0.10-0.30, and the mass ratio of the compound of Formula I to the lubricant is 1:0.01-0.10. Preferably, when a corresponding auxiliary material is selected, the amount of the auxiliary material is: the mass ratio of the compound of Formula I to the filler is 1:1.5-2.0, the mass ratio of the compound of Formula I to the binder is 1:0.02-0.04, the mass ratio of the compound of Formula I to the glidant is 1:0.012-0.020, the mass ratio of the compound of Formula I to the disintegrant is 1:0.15-0.25, and the mass ratio of the compound of Formula I to the lubricant is 1:0.02-0.06. The same technical effect can be achieved in any small ranges within the aforementioned preferred ratio ranges. For example, one aforementioned preferred ratio range of the lubricant can be 1:0.02-0.05. More preferably, when a corresponding auxiliary material is selected, the amount of the auxiliary material is: the mass ratio of the compound of Formula I to the filler is 1:1.75, the mass ratio of the compound of Formula I to the binder is 1:0.03, the mass ratio of the compound of Formula I to the glidant is 1:0.015, the mass ratio of the compound of Formula I to the disintegrant is 1:0.18 or 1:0.24, and the mass ratio of the compound of Formula I to the lubricant is 1:0.03 or 1:0.06.

Since the active ingredients of oral preparations can only be absorbed by the body when they are dissolved in the body, an oral preparation needs to undergo *in vivo and in vitro* dissolution tests after being prepared. There is a certain correlation between the *in vivo* and *in vitro* dissolution characteristics of drugs. The *in vitro* dissolution characteristics of drugs can reflect the dissolution behavior of the preparations *in vivo.* Therefore, a dissolution detection method with strong discrimination ability can be used to objectively evaluate the preparation quality of the preparations, and distinguish the quality differences of preparations from aspects such as the physicochemical properties of active ingredient, preparation formulations and preparation processes, thereby improving the safety and clinical efficacy of drugs. In pharmaceutical compositions for oral administration such as tablets, the dissolution properties of the active ingredients has a significant impact on their efficacy and safety, and standards regarding dissolution properties have been established in various countries. For instance, the methods of dissolution test are recorded in the pharmacopoeias of China, Japan, the United States and European countries, and various dissolution test solutions (hereinafter, also referred to as test solutions or dissolution solutions) are used in the dissolution test. These dissolution test solutions are adjusted to a pH range of 1 to 8. For instance, as the dissolution test solutions recorded in the pharmacopoeias of various countries, the Chinese Pharmacopoeia (2020 Edition, Part IV, 0931) shows the dissolution amount in the buffer solution, which is 250 mL of 0.2 mol/L sodium phosphate solution (adjust the pH value to 6.8 with 2 mol/L hydrochloric acid solution or 2 mol/L sodium hydroxide solution if necessary); the determination method in the Japanese Pharmacopoeia shows strongly acidic dissolution test solutions (e.g., the first solution recorded in the Japanese Pharmacopoeia, 0.1 N hydrochloric acid aqueous solution, etc.), dissolution test solutions with a pH of 3-5 (e.g., acetic acid-sodium acetate buffer, McIlvaine buffer, etc.) and dissolution test solutions with a pH of 6.8 (e.g., the second solution recorded in the Japanese Pharmacopoeia, phosphate buffer solution with a pH of 6.8, etc.), as well as water, etc. The following requirements are applied to oral preparations: when these dissolution test solutions used to conduct the dissolution tests, they should have good dissolution properties. Although the pharmacopoeias of various countries have disclosed similar dissolution media currently, the dissolution time and corresponding dissolution rate have not been disclosed for specific compositions.

The present disclosure provides a pharmaceutical composition against SARS-CoV-2, which exhibits a dissolution rate of 70.0% or more at 60 min in a phosphate buffer solution with a pH of 6.8 as a dissolution medium; further, which exhibits a dissolution rate of 43.0% or more at 15 min in a phosphate buffer solution with a pH of 6.8 as a dissolution **medium;** furthermore, which exhibits a dissolution rate of 60.0% or more at 30 min in a phosphate buffer solution with a pH of 6.8 as a dissolution medium. Moreover, the condition of a phosphate buffer solution with a pH of 6.8 provides a certain quality discrimination ability for the finished preparations.

These dissolution test solutions can be prepared by using the methods recorded in the pharmacopoeias of various countries. When the dissolution test solution is a buffer solution, the pH of these dissolution test solutions is

preferably within ±0.05 of the pH specified for each dissolution test solution.

In this specification, the determination in the dissolution test can be conducted in accordance with the dissolution and release determination method (Chinese Pharmacopoeia 2020 Edition, Part IV, General Chapter 0931, Method 2), in which 900 ml of phosphate buffer solution with a pH of 6.8 (mixing 250 ml of 0.2 mol/L potassium dihydrogen phosphate solution with 112 ml of 0.2 mol/L sodium hydroxide solution, then diluting to 1000 ml with water and mixing well) is used as the dissolution medium, the operations are conducted according to rules at a rotational speed of 75 rpm, and samples are taken after a certain period of time.

The present disclosure also provides a pharmaceutical composition against SARS-CoV-2 and a preparation method therefor, the effects of which can be further achieved by controlling the moisture content. Among them, the inventors surprisingly found that moisture not only affects the quality of the preparations during the preparation process, such as the formation of tablets, but also further affects the performance of the preparations, such as dissolution. Specifically, due to the inherent properties of the compound of Formula I, when the moisture content is too high, a "caking" phenomenon gradually occurs inside during storage, which in turn affects the dissolution of the preparations and the stability of long-term storage. However, in the pharmacopoeias of China, the United States, Japan, and the United Kingdom, there are no requirements for the moisture content in tablets. Therefore, the composition of the present disclosure has a more stringent control over moisture content than ordinary preparations. Specifically, the moisture content does not exceed 5.0%. Preferably, the moisture content of the composition is 1.0% to 5.0%, and more preferably 3.0% to 5.0%. Under such conditions, the composition containing the compound of Formula I of the present disclosure is very stable under long-term storage conditions or under accelerated storage conditions at 40°C/75% relative humidity. For example, when the composition of the present disclosure is stored at 40°C ± 2°C and 75% RH ± 5% RH for 3 months, the dissolution change of the composition is relatively small.

As used herein, "moisture content" refers to the total amount of moisture present in the composition. The moisture includes not only the unbound water or "moisture" of any excipient and/or any coating that may exist as the raw material, but also the bound water that is part of the crystal structure of any excipient and/or any coating that may exist as the raw material. The percentage of moisture content can be readily determined by those skilled in the art using the Karl Fischer technique. Furthermore, when calculating the moisture content of the composition of the present disclosure, the moisture content will never exceed 5.0% for the composition of the present disclosure.

**In** the present disclosure, the moisture content of the composition of the present disclosure can be determined by using the moisture determination method (Chinese Pharmacopoeia 2020 Edition, Part IV, General Chapter 0832, Method 1 (Karl Fischer Method)). The present disclosure provides a pharmaceutical composition against SARS-CoV-2 and a preparation method therefor, the effects of which can be achieved further through specific preparation technologies. In view of the inherent properties of the active ingredient and the auxiliary material, choosing a specific preparation technology can be conducive to obtaining preparations with excellent quality and further obtaining preparations with good stability. The present disclosure adopts non-wet granulation preparation technology, which refers to a preparation method that does not use wet granulation processes, including dry granulation, fluidized bed one-step granulation and powder direct compression, etc. Due to the wet and heat sensitivity of the compound of Formula **I,** the non-wet granulation preparation technology, preferably dry granulation, is adopted, which can avoid the instability of the active ingredient and the increase of impurities caused by the addition of granulation solution during the wet granulation process as well as the drying process after granulation.

The present disclosure provides a pharmaceutical composition against SARS-CoV-2 and a preparation method therefor, the effects of which can be further achieved by controlling the particle size of the active ingredient of the compound of Formula I. The particle size of the active ingredient may affect the feasibility of the preparation process and the dissolution of the product, and ultimately affect the dissolution *in vivo.* Therefore, it is necessary to study and control the particle size distribution of the active ingredient. Generally, as the particle size decreases, the particle size becomes fine and uniform, the specific surface area increases, the porosity rises, the adsorption capacity enhances, the solubility improves, the affinity increases, the chemical reaction rate increases, and the dissolution rate of the drug is improved. If the particle size is too large, it is not conducive to the mixing with the auxiliary materials, the mixing uniformity of the preparations will be poor, and the subsequent release will also be affected. Therefore, an appropriate particle size of the active ingredient not only ensures uniform mixing between the active ingredient and the auxiliary material, facilitating the formation of the preparations, but also improves the dissolution performance. When the particle size of the compound of Formula I simultaneously meets the conditions of $D10 \leq 8\ \mu m$, $D50 \leq 25\ \mu m$, and $D90 \leq 160\ \mu m$, the particle size distribution of the dry granules after dry granulation will not be significantly affected, and the dissolution curves of the obtained preparations in various dissolution media (pH 1.0, pH 4.5, pH 6.8, and purified water) will not be significantly affected. Preferably, the particle size of the compound of Formula I is $D10 \leq 5\ \mu m$, $D50 \leq 20\ \mu m$, and $D90 \leq 150\ \mu m$. More preferably, the particle size of the compound of Formula I is $D10 \leq 3\ \mu m$, $D50 \leq 15\ \mu m$, and $D90 \leq 140\ \mu m$.

The present disclosure provides a pharmaceutical composition against SARS-CoV-2 and a preparation method therefor, the effects of which can be further achieved by specific crystal forms, i.e., the crystal form of the compound of Formula I. Further, the crystal form of the compound of Formula I can optimize the dissolution performance of the obtained

preparations, enabling it to achieve better therapeutic effects in clinical treatment. The quality and efficacy of solid oral preparations depend on the selection of active ingredient, formulation design, and the production process of preparations. However, it is difficult to predict and control the processing-induced phase changes in the production process of preparations. Many drugs exist in polymorphic forms. Different crystal forms may have different physicochemical properties such as crystal habit, melting point, density, stability, solubility, and dissolution rate, as well as different powder properties such as bulk density, fluidity, and compressibility. Therefore, the crystal form of the drug may affect the production process of the preparations and the *in vivo* and *in vitro* drug effects of the preparations. Preferably, the crystal form of the compound of Formula I used in the present disclosure is the amorphous form of the compound, which is stable in nature, has good solubility and high dissolution rate, and is suitable for the development of preparations. Specifically, there are no sharp diffraction peaks in the X-ray powder diffraction pattern of the amorphous form. Further, there are two characteristic peaks at 2θ angles of 5°-15° and 15°-25° in the X-ray powder diffraction pattern of the amorphous form. Further, the X-ray powder diffraction pattern of the amorphous form is shown in FIG. 1.

The present disclosure provides a pharmaceutical composition against SARS-CoV-2 and a preparation method therefor, the effects of which can be achieved by controlling the mass ratio of the fillers, lactose and microcrystalline cellulose. Further, controlling the mass ratio of the fillers, lactose and microcrystalline cellulose, can optimize the dissolution performance of the obtained preparations, enabling it to achieve better therapeutic effects in clinical treatment. Based on the physicochemical properties of the active ingredient and the compatibility test results of the auxiliary materials, a mixture of lactose and microcrystalline cellulose was selected as the filler. If the amount of microcrystalline cellulose is too large, a "bottom attaching" phenomenon will occur during the dissolution process. That is, in the later stage of dissolution, microcrystals will accumulate at the bottom of the dissolution cup, resulting in incomplete drug release and adversely affecting drug dissolution. To ensure the preparation feasibility and performance of the obtained preparations, the mass ratio of lactose to microcrystalline cellulose is 1:0.5-5. Preferably, the mass ratio of lactose to microcrystalline cellulose is 1:0.6-3. The same technical effect can be achieved in any small ranges within the aforementioned preferred ratio ranges. For example, a preferred ratio range of the fillers can be 1:1-3. More preferably, the mass ratio of lactose to microcrystalline cellulose is 1:0.69, 1:0.98 or 1:2.

The present disclosure provides a pharmaceutical composition against SARS-CoV-2, and the tableting performance of the preparations can basically be achieved according to the above formulation composition. Although a very few tablets occasionally show loose or cracked phenomena, it is still within the reasonable production error range. To further reduce errors, the present disclosure can be further achieved by controlling the addition method and amount of the disintegrant and lubricant during the preparation process. Further, the addition method and amount of the disintegrant and lubricant during the preparation process can optimize the dissolution performance of the obtained preparations, enabling it to achieve better therapeutic effects in clinical treatment. When the disintegrant is intra-granularly added, the disintegration effect starts from the inside of the granules, causing them to completely disintegrate. Since the disintegrant is encapsulated within the granules, its contact with water is delayed, and the disintegrant has already been exposed to mild heat during the granulation process, so the disintegration effect is relatively weak. When the disintegrant is added extra-granularly, the disintegration rate is relatively fast, but the disintegration effect mainly occurs between granules, and after disintegration, the materials are often in a granular state rather than a fine powder. The lubricant is added both intra-granularly and extra-granularly to ensure the fluidity between the separated granules while improving the friction between the polymer and the hot metal surface of the processing equipment, thus guaranteeing the quality of tableting.

With regard to the pharmaceutical composition of the present disclosure, the disintegrant is added both intra-granularly and extra-granularly during the preparation, and the lubricant is also added both intra-granularly and extra-granularly during the preparation. When the disintegrant is added both intra-granularly and extra-granularly during the preparation, the mass ratio of the intragranular disintegrant to the extragranular disintegrant is 1:0.5-3, the dissolution performance of the drug is good, and there is no significant difference in various quality attributes (particle size, angle of repose, bulk density, tap density) among the granules (tablets) of the preparations, nor is there a significant difference in dissolution rate, preferably, the mass ratio of the intragranular disintegrant to the extragranular disintegrant is 1:0.8-2, more preferably, the mass ratio of the intragranular disintegrant to the extragranular disintegrant is 1:1. When the lubricant is added both intra-granularly and extra-granularly during the preparation, the mass ratio of the intragranular lubricant to the extragranular lubricant is 1:0.5-3, similarly, the dissolution performance of the drug is good, and there is no significant difference in various quality attributes (particle size, angle of repose, bulk density, tap density) among the granules (tablets) of the preparations, nor is there a significant difference in dissolution rate, preferably, the mass ratio of the intragranular lubricant to the extragranular lubricant is 1:0.8-2, more preferably, the mass ratio of the intragranular lubricant to the extragranular lubricant is 1:1.

According to the actual production situation of the preparations, it is well known to those skilled in the art that the mass ratio between auxiliary materials is allowed to have a certain error range. The error range of the mass ratio described in the present disclosure can be ±0.5%.

The present disclosure also provides a composition below including the following components. This formulation is the preferred technical solution of the present disclosure, which simultaneously solves the problems related to the

preparation stability of the compound of Formula I, the preparation process (sticking, etc.) and dissolution. The obtained preparations have excellent quality, with no problems such as sticking, loosening or cracking, and has good dissolution performance and excellent stability, which are in line with the medicinal effects.

A pharmaceutical composition, including the following components,

| Name | Compound of Formula I: auxiliary material (mg/mg) | Amount, mg/tablet |
|---|---|---|
| Compound of Formula I | - | 200.0 |
| Lactose | 1:0.582 | 116.4 |
| Microcrystalline cellulose | 1:1.163 | 232.6 |
| Hydroxypropyl methylcellulose | 1:0.03 | 6.0 |
| Colloidal silica | 1:0.015 | 3.0 |
| Cross-linked sodium carboxymethyl cellulose (intragranular) | 1:0.09 | 18.0 |
| Magnesium stearate (intragranular) | 1:0.015 | 3.0 |
| Cross-linked sodium carboxymethyl cellulose (extragranular) | 1:0.09 | 18.0 |
| Magnesium stearate (extragranular) | 1:0.015 | 3.0 |

The compound of Formula I has a particle size of D10 ≤ 3 μm, D50 ≤ 15 μm, D90 ≤ 140 μm, and the compound of Formula I is in amorphous form.

A pharmaceutical composition, including the following components,

| Name | Compound of Formula I: auxiliary material (mg/mg) | Amount, mg/tablet |
|---|---|---|
| Compound of Formula I | - | 200.0 |
| Lactose | 1:0.884 | 176.8 |
| Microcrystalline cellulose | 1:0.866 | 173.2 |
| Hydroxypropyl methylcellulose | 1:0.03 | 6.0 |
| Colloidal silica | 1:0.015 | 3.0 |
| Cross-linked sodium carboxymethyl cellulose (intragranular) | 1:0.09 | 18.0 |
| Magnesium stearate (intragranular) | 1:0.015 | 3.0 |
| Cross-linked sodium carboxymethyl cellulose (extragranular) | 1:0.09 | 18.0 |
| Magnesium stearate (extragranular) | 1:0.015 | 3.0 |

, the compound of Formula I has a particle size of D10 ≤ 3 μm, D50 ≤ 15 μm, D90 ≤ 140 μm, and the compound of Formula I is in amorphous form.

A pharmaceutical composition, including the following components,

| Name | Compound of Formula I: auxiliary material (mg/mg) | Amount, mg/tablet |
|---|---|---|
| Compound of Formula I | - | 200.0 |
| Lactose | 1:1.032 | 206.4 |
| Microcrystalline cellulose | 1:0.713 | 142.6 |
| Hydroxypropyl methylcellulose | 1:0.03 | 6.0 |
| Colloidal silica | 1:0.015 | 3.0 |
| Cross-linked sodium carboxymethyl cellulose (intragranular) | 1:0.12 | 24.0 |

(continued)

| Name | Compound of Formula I: auxiliary material (mg/mg) | Amount, mg/tablet |
|---|---|---|
| Magnesium stearate (intragranular) | 1:0.03 | 6.0 |
| Cross-linked sodium carboxymethyl cellulose (extragranular) | 1:0.12 | 24.0 |
| Magnesium stearate (extragranular) | 1:0.03 | 6.0 |

, the compound of Formula I has a particle size of D10 ≤ 3 μm, D50 ≤ 15 μm, D90 ≤ 140 μm, the compound of Formula I is in amorphous form.

Further, a pharmaceutical composition, including the following components,

| Name | Mass (parts) |
|---|---|
| Compound of Formula I | 1 |
| Lactose | 0.582 |
| Microcrystalline cellulose | 1.163 |
| Hydroxypropyl methylcellulose | 0.03 |
| Colloidal silica | 0.015 |
| Cross-linked sodium carboxymethyl cellulose (intragranular) | 0.09 |
| Magnesium stearate (intragranular) | 0.015 |
| Cross-linked sodium carboxymethyl cellulose (extragranular) | 0.09 |
| Magnesium stearate (extragranular) | 0.015 |

The compound of Formula I has a particle size of D10 ≤ 3 μm, D50 ≤ 15 μm, D90 ≤ 140 μm, the compound of Formula I is in amorphous form, and the pharmaceutical composition has a moisture content of 3.0% to 5.0%.

Further, a pharmaceutical composition, including the following components,

| Name | Mass (parts) |
|---|---|
| Compound of Formula I | 1 |
| Lactose | 0.884 |
| Microcrystalline cellulose | 0.866 |
| Hydroxypropyl methylcellulose | 0.03 |
| Colloidal silica | 0.015 |
| Cross-linked sodium carboxymethyl cellulose (intragranular) | 0.09 |
| Magnesium stearate (intragranular) | 0.015 |
| Cross-linked sodium carboxymethyl cellulose (extragranular) | 0.09 |
| Magnesium stearate (extragranular) | 0.015 |

The compound of Formula I has a particle size of D10 ≤ 3 μm, D50 ≤ 15 μm, D90 ≤ 140 μm, the compound of Formula I is in amorphous form, and the pharmaceutical composition has a moisture content of 3.0% to 5.0%.

Further, a pharmaceutical composition, including the following components,

| Name | Mass (parts) |
|---|---|
| Compound of Formula I | 1 |
| Lactose | 1.032 |
| Microcrystalline cellulose | 0.713 |

(continued)

| Name | Mass (parts) |
|---|---|
| Hydroxypropyl methylcellulose | 0.03 |
| Colloidal silica | 0.015 |
| Cross-linked sodium carboxymethyl cellulose (intragranular) | 0.12 |
| Magnesium stearate (intragranular) | 0.03 |
| Cross-linked sodium carboxymethyl cellulose (extragranular) | 0.12 |
| Magnesium stearate (extragranular) | 0.03 |

The compound of Formula I has a particle size of D10 ≤ 3 μm, D50 ≤ 15 μm, D90 ≤ 140 μm, the compound of Formula I is in amorphous form, and the pharmaceutical composition has a moisture content of 3.0% to 5.0%.

Further, a pharmaceutical composition, including the following components,

| Name | Mass (parts) |
|---|---|
| Compound of Formula I | 1 |
| Lactose | 0.582 |
| Microcrystalline cellulose | 1.163 |
| Hydroxypropyl methylcellulose | 0.03 |
| Colloidal silica | 0.015 |
| Cross-linked sodium carboxymethyl cellulose (intragranular) | 0.09 |
| Magnesium stearate (intragranular) | 0.015 |
| Cross-linked sodium carboxymethyl cellulose (extragranular) | 0.09 |
| Magnesium stearate (extragranular) | 0.015 |

The compound of Formula I has a particle size of D10 ≤ 3 μm, D50 ≤ 15 μm, D90 ≤ 140 μm, the compound of Formula I is in amorphous form, and the pharmaceutical composition exhibits a dissolution rate of 70.0% or more at 60 min in a phosphate buffer solution with a pH of 6.8 as a dissolution medium.

Further, a pharmaceutical composition, including the following components,

| Name | Mass (parts) |
|---|---|
| Compound of Formula I | 1 |
| Lactose | 0.884 |
| Microcrystalline cellulose | 0.866 |
| Hydroxypropyl methylcellulose | 0.03 |
| Colloidal silica | 0.015 |
| Cross-linked sodium carboxymethyl cellulose (intragranular) | 0.09 |
| Magnesium stearate (intragranular) | 0.015 |
| Cross-linked sodium carboxymethyl cellulose (extragranular) | 0.09 |
| Magnesium stearate (extragranular) | 0.015 |

the compound of Formula I has a particle size of D10 ≤ 3 μm, D50 ≤ 15 μm, D90 ≤ 140 μm, the compound of Formula I is in amorphous form, and the pharmaceutical composition exhibits a dissolution rate of 70.0% or more at 60 min in a phosphate buffer solution with a pH of 6.8 as a dissolution medium.

Further, a pharmaceutical composition, including the following components,

| Name | Mass (parts) |
|---|---|
| Compound of Formula I | 1 |
| Lactose | 1.032 |
| Microcrystalline cellulose | 0.713 |
| Hydroxypropyl methylcellulose | 0.03 |
| Colloidal silica | 0.015 |
| Cross-linked sodium carboxymethyl cellulose (intragranular) | 0.12 |
| Magnesium stearate (intragranular) | 0.03 |
| Cross-linked sodium carboxymethyl cellulose (extragranular) | 0.12 |
| Magnesium stearate (extragranular) | 0.03 |

the compound of Formula I has a particle size of D10 ≤ 3 μm, D50 ≤ 15 μm, D90 ≤ 140 μm, the compound of Formula I is in amorphous form, and the pharmaceutical composition exhibits a dissolution rate of 70.0% or more at 60 min in a phosphate buffer solution with a pH of 6.8 as a dissolution medium.

Further, a pharmaceutical composition, including the following components,

| Name | Mass (parts) |
|---|---|
| Compound of Formula I | 1 |
| Lactose | 0.582 |
| Microcrystalline cellulose | 1.163 |
| Hydroxypropyl methylcellulose | 0.03 |
| Colloidal silica | 0.015 |
| Cross-linked sodium carboxymethyl cellulose (intragranular) | 0.09 |
| Magnesium stearate (intragranular) | 0.015 |
| Cross-linked sodium carboxymethyl cellulose (extragranular) | 0.09 |
| Magnesium stearate (extragranular) | 0.015 |

The compound of Formula I has a particle size of D10 ≤ 3 μm, D50 ≤ 15 μm, D90 ≤ 140 μm, the compound of Formula I is in amorphous form, and the pharmaceutical composition exhibits a dissolution rate of 70.0% or more at 60 min in a phosphate buffer solution with a pH of 6.8 as a dissolution medium.

Further, a pharmaceutical composition, including the following components,

| Name | Mass (parts) |
|---|---|
| Compound of Formula I | 1 |
| Lactose | 0.884 |
| Microcrystalline cellulose | 0.866 |
| Hydroxypropyl methylcellulose | 0.03 |
| Colloidal silica | 0.015 |
| Cross-linked sodium carboxymethyl cellulose (intragranular) | 0.09 |
| Magnesium stearate (intragranular) | 0.015 |
| Cross-linked sodium carboxymethyl cellulose (extragranular) | 0.09 |
| Magnesium stearate (extragranular) | 0.015 |

The compound of Formula I has a particle size of D10 ≤ 3 μm, D50 ≤ 15 μm, D90 ≤ 140 μm, the compound of

Formula I is in amorphous form, and the pharmaceutical composition exhibits a dissolution rate of 70.0% or more at 60 min in a phosphate buffer solution with a pH of 6.8 as a dissolution medium.

Further, a pharmaceutical composition, including the following components,

| Name | Mass (parts) |
|---|---|
| Compound of Formula I | 1 |
| Lactose | 1.032 |
| Microcrystalline cellulose | 0.713 |
| Hydroxypropyl methylcellulose | 0.03 |
| Colloidal silica | 0.015 |
| Cross-linked sodium carboxymethyl cellulose (intragranular) | 0.12 |
| Magnesium stearate (intragranular) | 0.03 |
| Cross-linked sodium carboxymethyl cellulose (extragranular) | 0.12 |
| Magnesium stearate (extragranular) | 0.03 |

The compound of Formula I has a particle size of D10 ≤ 3 μm, D50 ≤ 15 μm, D90 ≤ 140 μm, the compound of Formula I is in amorphous form, and the pharmaceutical composition exhibits a dissolution rate of 70.0% or more at 60 min in a phosphate buffer solution with a pH of 6.8 as a dissolution medium.

The second object of the present disclosure is to provide a preparation method of the pharmaceutical composition, which is a non-wet granulation preparation technology.

The third object of the present disclosure is to provide a pharmaceutical tablet, which includes a core and a coating covering the core, the core is composed of the pharmaceutical composition of the present disclosure.

According to the different coating materials, coated tablets can mainly be classified into sugar-coated tablets, film-coated tablets and enteric-coated tablets. In the present disclosure, the coating is a suitable coating known to have no negative impact on the dissolution rate of the final preparations, preferably a gastric-soluble film coating. The film coating can provide the tablet core with a sealed coating, thereby protecting patients and clinical personnel and preventing the tablet core from coming into contact with air and moisture, thus reducing the chance of drug degradation.

Suitable film coating materials include film-forming agents, such as film-forming polymers. Preferably, the film coating materials also include additional components, such as plasticizers, colorants, dispersing aids and light-screening agents. Plasticizers can be used to improve the film flexibility, durability and adhesion properties of the film coating. The preferred film-forming polymers are selected from one or more of film-forming vinyl polymers (e.g., polyvinyl alcohol), film-forming acrylic polymers (e.g., methacrylic acid-methyl methacrylate copolymer), water-soluble cellulose ethers (e.g., hydroxypropyl methylcellulose), etc. The preferred plasticizers are selected from glycerol, acetylated monoglycerides, citrates, propylene glycol, polyethylene glycol, triglycerides or phthalates. Suitable light-screening agents and colorants include, for example, titanium dioxide, yellow iron oxide, black iron oxide and fancy red aluminum lake. Suitable dispersing aids include, for example, talc.

Suitable film coating materials can be concentrates, which are formulated with water or organic solvents into coating solutions before being sprayed onto the tablet core. In the present disclosure, the coating material used is a medicinal film coating premix (gastric-soluble type) (Opadry 85F64732-CN pink), which is formulated into a coating solution with a solid content of 15% and sprayed onto the tablet core. Among them, the mass of the coating is 0.5% to 10% of the mass of the tablet core, preferably 1% to 6%, and more preferably 3%.

In the present disclosure, the tablet of the compound of Formula I has a specification of 100 mg - 300 mg, e.g., 100 mg, 150 mg, 200 mg, 250 mg, 300 mg, preferably 150 mg - 250 mg, more preferably 200 mg.

According to the present disclosure, also provided is a preparation method of the above pharmaceutical tablet, which specifically includes the following steps:

1) sizing the prescribed amounts of the compound of Formula I and lactose through a sieve, then mixing and screening the prescribed amounts of hydroxypropyl methylcellulose, cross-linked sodium carboxymethyl cellulose (intragranular) and colloidal silica, and finally screening the prescribed amount of microcrystalline cellulose, where the specification of the sieve is: Φ1.0 mm, and the frequency for the sizing is 8 Hz,

premixing 1 the screened materials at a rotational speed of 10 rpm for 15 min to obtain a premix 1,

at the end of the premixing 1, adding magnesium stearate (intragranular) and premixing 2 at a rotational speed of 10 rpm for 5 min to obtain a premix 2.

2) putting the pre-mixture 2 into a dry granulation machine for dry granulation, where the feeding speed of the hopper is 5-10 rpm, the feeding speed of the screw is 10-20 rpm, the speed of the pressure wheel is 13-23 rpm, the hydraulic pressure is 60-80 bar, the gap of the pressure wheel is 1.0 mm, the sieve specification for the sizing 1 is 10 mesh, and the speed for the sizing 1 is 30-80 rpm, the sieve specification for the sizing 2 is 24 mesh, and the speed for the sizing 2 is 80-180 rpm. After sizing, dry granules are obtained. After the dry granulation is completed, samples are taken for the detection of drying weight loss, which shall not exceed 5.0%.

3) transferring the dry granules along with the calculated cross-linked sodium carboxymethyl cellulose (extragranular) to a mixer and pre-mix at a mixing speed of 10 rpm for 5 min to obtain a premix; then adding the calculated magnesium stearate (extragranular) and mixing with the premix in the mixer at a mixing speed of 10 rpm for 3 min to obtain a total mixture. After the total mixing is completed, samples are taken for the inspection of intermediate products.

4) after the content of the total mixed granules is inspected to be qualified, tableting under the following conditions: the die specification is 17 mm * 8.5 mm shallow concave punch, the tableting speed is 100-180 thousand tablets per hour, the main pressure of the tablet press is 8-11 kN, and the hardness is controlled within the range of 100-180 N. After the tableting is completed, samples are taken for the inspection of intermediate products.

5) weighing the film coating premix and purified water to formulate a coating solution, and coating using a high-efficiency coating machine under the following conditions: the inlet air temperature for coating is 40-66°C, the outlet air temperature is 35-55°C, the material temperature is 35-55°C, the liquid spraying speed is 0.1-0.5 kg/min, the atomization pressure is 1.0-5.0 bar, and the weight gain range of coating is 2.0-4.0%. After the coating is completed, samples are taken for the inspection of appearance, coating weight gain and drying weight loss, which shall not exceed 5.0%.

The fourth object of the present disclosure is to provide a use of the pharmaceutical tablet in the preparation of a product for preventing, alleviating or treating an infection or a disease caused by SARS-CoV-2. The SARS-CoV-2 includes both unvariants and variants of the SARS-CoV-2. Variants of the SARS-CoV-2 include those selected from the Alpha variant, Beta variant, Gamma variant, Delta variant, Lambda variant and/or Omicron variant of the SARS-CoV-2. Infections include fever, cough, sore throat, pneumonia, acute respiratory tract infection, severe acute respiratory tract infection, hypoxic respiratory failure and acute respiratory distress syndrome, sepsis or septic shock. Preferably, the disease includes COVID-19.

The preparation method of the pharmaceutical composition containing the compound of Formula I and the tablet containing it provided by the present disclosure is simple and has the feasibility of preparation process, the preparations have excellent properties (no sticking, cracking, etc.), good stability and good dissolution, and exhibit a dissolution rate of 70.0% or more at 60 min. Furthermore, under long-term storage conditions or under accelerated storage conditions, the preparations also have excellent stability and dissolution, and the dissolution meets the pharmaceutical standards.

## BRIEF DESCRIPTION OF THE DRAWINGS

FIG. 1 shows an XRPD spectrogram with Cu-Kα radiation of the amorphous form of the compound of Formula I;

FIG. 2 shows an XRPD spectrogram with Cu-Kα radiation of the crystal form I of the compound of Formula I;

FIG. 3 shows the dissolution curve graphs of Example 2 and Comparative Example 6 under the condition of a phosphate buffer solution with a pH of 6.8;

FIG. 4 shows the dissolution curve graphs of Examples 2, 6, 7 and Comparative Example 5.

## DETAILED DESCRIPTION

The present disclosure will be further described in detail in combination with the examples.

The following examples are preferred embodiments of the present disclosure, but the embodiments of the present disclosure are not limited by the above examples. Any other changes, modifications, substitutions, combinations,

and simplifications made without deviating from the spirit and principle of the present disclosure shall be equivalent replacement methods and are all included within the protection scope of the present disclosure.

Determination of dissolution rate

The dissolution rate was determined according to the dissolution and release determination method (Chinese Pharmacopoeia 2020 Edition, Part IV, General Chapter 0931, Method 2).

Dissolution conditions: Take 900 ml of phosphate buffer solution with a pH of 6.8 (mixing 250 ml of 0.2 mol/L potassium dihydrogen phosphate solution with 112 ml of 0.2 mol/L sodium hydroxide solution, then diluting to 1000 ml with water and mixing well) as the dissolution medium, operate at a speed of 75 rpm, and take samples after a certain period of time.

Dissolution tester: Raytor RT-612-AT

Test solution: Take an appropriate amount of the dissolution solution, filter the dissolution solution, discard 2 ml of the initial filtrate, and take the subsequent filtrate.

Control solution: Take an appropriate amount of the commercially available Leritrelvir tablets as control, accurately weigh the tablets, dissolve the tablets in the solvent [anhydrous ethanol-water (80:20)], and quantitatively dilute the solution to prepare a solution containing about 0.22 mg (0.2 g specification) or 0.11 mg (0.1 g specification) per 1ml.

The chromatographic conditions and system suitability requirements were determined in accordance with the high-performance liquid chromatography method (Chinese Pharmacopoeia 2020 Edition, Part IV, General Chapter 0512).

Solvent: absolute ethanol-water (80:20).

Test solution: Take 10 tablets of this product, accurately weigh the tablets, grind into fine powder, and accurately weigh an appropriate amount of the fine powder (approximately equivalent to 200 mg of Leritrelvir), place the powder in a 100 ml volumetric flask, add an appropriate amount of a solvent and sonicate to dissolve the powder, allow the solution to cool to room temperature, dilute the solution with the solvent to the mark, shake well, centrifuge at 8000 rpm for 10 min, accurately measure an appropriate amount of the supernatant, quantitatively dilute the supernatant with the solvent to prepare a solution containing about 0.2 mg per 1 ml.

Control solution: Take an appropriate amount of the commercially available Leritrelvir tablets as control, accurately weigh the tablets, dissolve the tablets in the solvent and quantitatively dilute the solution to prepare a solution containing about 0.2 mg per 1 ml.

Instrument: Agilent 1260 Infinity II VWD/DAD

Chromatographic conditions: octadecylsilane-bonded silica gel as the packing material (Waters Xbridge C18, 4.6 mm×150 mm, 3.5 μm or equivalent chromatographic column); 0.005 mol/L dipotassium hydrogen phosphate solution (adjusting the pH value to 8.4 with 1% phosphoric acid solution) - methanol (30:70) as the mobile phase, with a flow rate of 1.0 ml/min; the detection wavelength is 210 nm; the column temperature is 55°C, and the injection volume is 10 μl.

System suitability requirements: in the chromatogram of the control solution, the theoretical number of plates, calculated based on the peak of Leritrelvir, should not be less than 2000.

Determination method: Accurately measure the test solution and the control solution and inject them into the liquid chromatograph respectively, record the chromatogram, and calculate using the peak area according to the external standard method. Calculation formula:

$$\text{Content of test product\%} = \frac{\dfrac{\text{Weighed amount of the control product} \times (\text{Content of the control product} + \text{Content of isomers in the control product})}{\text{Dilution ratio of the control product} \times \text{Peak area of the control product}} \times \dfrac{\text{Peak area of the test product} \times (100\% - \text{Content of isomers in the test product})}{\dfrac{\text{Weighed amount of the test product} \times \text{Specification of the product}}{\text{Dilution ratio of the test product} \times \text{Average piece weight}}}}{} \times 100\%$$

Moisture determination method:

1. Instrument and Equipment

(1) Moisture meter: Metrohm 915KF Ti-Touch

(2) Electronic analytical balance: Sartorius BCE224I-1CCN

2. Reagents and Control

(1) Ultrapure water/purified water: home-made

(2) Absolute methanol, purchased from Chengdu Kelong Chemical Co., Ltd.

(3) Pyridine-free Karl Fischer reagent, purchased from Tianjin Kermel Chemical Reagent Co., Ltd

3. Operating steps

Operation in accordance with the moisture determination method, Method 1 (Karl Fischer Method) in Chinese Pharmacopoeia 2020 Edition, Part IV, General Chapter 0832. Calibration of Fischer test solution: Accurately weigh about 10 mg of purified water and directly calibrate three times with a moisture meter. Determination of the test product: Take the product, grind finely, weigh about 200 mg of fine powder, and determine the moisture twice or more with the moisture meter.

The determination results can be expressed as range values or as averages of several measurements, but in any case, the averages of several measurements shall all be within the range values of the current test.

The amorphous form of the compound of Formula I can be prepared and detected with reference to PCT/CN2023/120437;

the crystal form I of the compound of Formula I can be prepared and detected with reference to PCT/CN2023/120726;
the compounds of Formula I above are all from Guangdong Raynovent Biotech Co., Ltd.
The particle size of the required active ingredient was obtained by controlling the size of the crushing screen.

The particle size of the active ingredient of the compound of Formula I was tested under the following conditions:

Instrument name: Malvern laser particle size analyzer;
Instrument model: Mastersizer 3000;
Sample injector: dry method;
Injection rate: 35%;
Dispersion air pressure: 2 bar;
Measuring time: 5 s.

Example 1: Compatibility test of active ingredient and auxiliary materials

The selected auxiliary materials were lactose, microcrystalline cellulose, mannitol, cross-linked sodium carboxymethyl cellulose, sodium stearyl fumarate, poloxamer, hydroxypropyl methylcellulose, glyceryl behenate, colloidal silica, sodium dodecyl sulfate, magnesium stearate and film coating premix. The auxiliary materials were respectively mixed with the main drug in the proportions shown in Table 1 and then placed according to the conditions in Table 1. Samples were taken at specified time points to examine the changes in properties, content, and relevant substances (total impurities). The analytical method adopted was the HPLC method.

Table 1: Compatibility test results of auxiliary materials

| Group | Investigated items | Day 0 | 60°C (open) | | Room temperature/92.5% RH (open) | | Illumination test |
|---|---|---|---|---|---|---|---|
| | | | Day 5 | Day 10 | Day 5 | Day 10 | Day 10 |
| Compound of Formula I, the active ingredient | characteristics | white powder | white powder | white powder | white powder | white powder | white powder |
| | content% | 100.0 | 100.5 | 100.0 | 101.9 | 106.7 | 56.8 |
| | total impurities% | 0.44 | 0.50 | 0.82 | 0.29 | 0.32 | 21.35 |
| Compound of Formula I, the active ingredient: lactose=1:5 | characteristics | white powder | white powder | white powder | white powder | white powder | white powder |
| | content% | 106.7 | 100.2 | 100.8 | 100.9 | 102.5 | 47.1 |
| | total impurities% | 0.59 | 0.70 | 0.65 | 0.55 | 0.27 | 27.16 |
| Compound of Formula I, the active ingredient: microcrystalline cellulose=1:5 | characteristics | white powder | white powder | white powder | white powder | white powder | white powder |
| | content% | 101.4 | 101.6 | 101.1 | 101.9 | 102.8 | 43.6 |
| | total impurities% | 0.43 | 0.55 | 0.98 | 0.52 | 0.45 | 17.17 |
| Compound of Formula I, the active ingredient: mannitol =1:5 | characteristics | white powder | white powder | white powder | white powder | white powder | white powder |
| | content% | 100.1 | 103.0 | 101.0 | 101.6 | 103.0 | 48.4 |
| | total impurities% | 0.43 | 0.68 | 1.07 | 0.57 | 0.56 | 26.19 |
| Compound of Formula I, the active ingredient: sodium carboxymethyl cellulose =1:1 | characteristics | white powder | white powder | white powder | white powder | white powder | white powder |
| | content% | 99.8 | 101.0 | 100.7 | 101.7 | 102.5 | 51.1 |
| | total impurities% | 0.52 | 0.77 | 0.94 | 0.47 | 0.48 | 24.00 |
| Compound of Formula I, the active ingredient: sodium stearyl fumarate=1:1 | characteristics | white powder | white powder | white powder | white powder | white powder | white powder |
| | content% | 99.1 | 102.3 | 100.8 | 103.6 | 102.7 | 65.8 |
| | total impurities% | 0.34 | 0.44 | 0.39 | 0.36 | 0.11 | 8.65 |
| Compound of Formula I, the active ingredient: poloxamer188=1:1 | characteristics | white powder | brown semi-solid | brown semi-solid | white crystal | white crystal | white powder |
| | content% | 94.3 | 9.5 | 13.4 | 94.5 | 80.7 | 53.5 |
| | total impurities% | 0.29 | 79.43 | 71.40 | 4.93 | 15.12 | 22.98 |
| Compound of Formula I, the active ingredient: hydroxypropyl methylcellulose=1:1 | characteristics | white powder | white powder | white powder | white powder | white powder | white powder |
| | content% | 93.5 | 102.7 | 100.0 | 103.5 | 101.4 | 61.8 |
| | total impurities% | 0.42 | 0.78 | 0.83 | 0.32 | 0.69 | 17.43 |

(continued)

| Group | Investigated items | Day 0 | 60°C (open) | | Room temperature/92.5% RH (open) | | Illumination test |
|---|---|---|---|---|---|---|---|
| | | | Day 5 | Day 10 | Day 5 | Day 10 | Day 10 |
| Compound of Formula I, the active ingredient: glyceryl behenate=1:1 | characteristics | white powder | white powder | white powder | white powder | white powder | white powder |
| | content% | 104.2 | 98.9 | 91.8 | 102.9 | 102.4 | 62.5 |
| | total impurities% | 0.54 | 1.84 | 5.26 | 0.36 | 0.40 | 17.80 |
| Compound of Formula I, the active ingredient: colloidal silica=1:1 | characteristics | white powder | white powder | white powder | white powder | white powder | white powder |
| | content% | 96.9 | 102.4 | 101.2 | 103.1 | 102.9 | 52.9 |
| | total impurities% | 0.44 | 0.84 | 1.10 | 0.52 | 0.63 | 22.92 |
| Compound of Formula I, the active | characteristics | white powder | white powder | white powder | white powder | white liquid | white powder |
| ingredient: sodium dodecyl sulfate=1:1 | content% | 98.1 | 103.5 | 101.0 | 93.9 | 82.5 | 61.0 |
| | total impurities% | 0.37 | 0.68 | 1.47 | 7.50 | 16.26 | 17.70 |
| Compound of Formula I, the active ingredient: magnesium stearate=2:1 | characteristics | white powder | white powder | white powder | white powder | white powder | white powder |
| | content% | 100.8 | 101.7 | 100.6 | 101.3 | 101.3 | 64.0 |
| | total impurities% | 0.54 | 0.54 | 1.03 | 0.54 | 0.74 | 15.73 |

It can be seen from the above results that regarding the characteristics, after the active ingredient of the compound of Formula I was placed with poloxamer 188 for 5 and 10 days under high-temperature conditions, the characteristics of the binary mixture changed, and the appearance characteristics changed from white powder to brown semi-solid; after the active ingredient of the compound of Formula I was placed with poloxamer 188 for 5 and 10 days under high-humidity conditions, the characteristics of the binary mixture changed, and the appearance characteristics changed from white powder to white crystals; after the active ingredient of the compound of Formula I was placed with sodium dodecyl sulfate for 10 days under high-humidity conditions, the appearance characteristics changed from white powder to white liquid; after the active ingredient of the compound of Formula I was placed with other auxiliary materials for 5 and 10 days respectively, there was no obvious change in appearance, all white powder.

Regarding the content, the content of the binary mixture of the active ingredient of the compound of Formula I with poloxamer 188 and glyceryl behenate decreased significantly after being placed under high-temperature conditions for 5 and 10 days; the content of the binary mixture of the active ingredient of the compound of Formula I with sodium dodecyl sulfate decreased significantly under high-humidity conditions; the content of the active ingredient of the compound of Formula I and all auxiliary materials decreased significantly under illumination conditions; and all the other binary mixtures showed no significant changes in the content compared with Day 0 under high-temperature and high-humidity conditions.

Regarding the relevant substances, the total impurities of the active ingredient of the compound of Formula I and poloxamer 188 significantly increased under high-temperature conditions; the total impurities of the active ingredient of compound I with poloxamer 188 and sodium dodecyl sulfate significantly increased under high-humidity conditions, respectively; the total impurities of each binary mixture of the active ingredient of the compound of Formula I with all auxiliary materials increased under illumination conditions, and the increase trend was basically consistent with that of the active ingredient of the compound of Formula I.

In conclusion, the active ingredient of the compound of Formula I has good compatibility with lactose, microcrystalline cellulose, mannitol, cross-linked sodium carboxymethyl cellulose, sodium stearyl fumarate, hydroxypropyl methylcellulose, colloidal silica and magnesium stearate, all of which can be applied in the research of the formulation of the preparations. However, its compatibility with poloxamer 188, sodium dodecyl sulfate and glyceryl behenate is relatively poor.

Example 2: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 116.4 |
| Microcrystalline cellulose | 232.6 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

Preparation method:

1) sizing the prescribed amounts of the compound of Formula I and lactose through a sieve, then mixing and screening the prescribed amounts of hydroxypropyl methylcellulose, cross-linked sodium carboxymethyl cellulose (intragranular) and colloidal silica, and finally screening the prescribed amount of microcrystalline cellulose, where the specification of the sieve is: Φ1.0 mm, and the frequency for the sizing is 8 Hz,

premixing 1 the screened materials in a mixer at a rotational speed of 10 rpm for 15 min to obtain a premix 1,

at the end of the premixing 1, adding magnesium stearate (intragranular) and premixing 2 at a rotational speed of 10 rpm for 5 min to obtain a premix 2.

2) putting the pre-mixture 2 into a dry granulation machine for dry granulation, where the feeding speed of the hopper is 5-10 rpm, the feeding speed of the screw is 10-20 rpm, the speed of the pressure wheel is 13-23 rpm, the hydraulic pressure is 60-80 bar, the gap of the pressure wheel is 1.0 mm, the sieve specification for the sizing 1 is 10 mesh, and the speed for the sizing 1 is 30-80 rpm, the sieve specification for the sizing 2 is 24 mesh, and the speed for the sizing 2 is 80-180 rpm. After sizing, dry granules are obtained. After the dry granulation is completed, samples are taken for the detection of drying weight loss, which shall not exceed 5.0%.

3) transferring the dry granules along with the calculated cross-linked sodium carboxymethyl cellulose (extragranular) to a mixer and pre-mix at a mixing speed of 10 rpm for 5 min to obtain a premix; then adding the calculated magnesium stearate (extragranular) and mixing with the premix in the mixer at a mixing speed of 10 rpm for 3 min to obtain a total mixture. After the total mixing is completed, samples are taken for the inspection of intermediate products.

4) after the content of the total mixed granules is inspected to be qualified, tableting under the following conditions: the die specification is 17 mm * 8.5 mm shallow concave punch, the tableting speed is 100-180 thousand tablets per hour, the main pressure of the tablet press is 8-11 kN, and the hardness is controlled within the range of 100 to 180 N. After the tableting is completed, samples are taken for the inspection of intermediate products.

5) weighing the film coating premix and purified water to formulate a coating solution, and coating using a high-efficiency coating machine under the following conditions: the inlet air temperature for coating is 40-66°C, the outlet air temperature is 35-55°C, the material temperature is 35-55°C, the liquid spraying speed is 0.1-0.5 kg/min, the atomization pressure is 1.0-5.0 bar, and the weight gain range of coating is 2.0-4.0%. After the coating is completed, samples are taken for the inspection of appearance, coating weight gain and drying weight loss, which shall not exceed 5.0%, and the moisture content is controlled within the range of 3.0% to 5.0% by adjusting the length of drying time.

Example 3: Preparation of tablets of the compound of Formula I

Formulation composition of 1000 tablets of the compound of Formula I

| Name | Amount (g) |
|---|---|
| Compound of Formula I (amorphous form, D10 ≤ 5 μm, D50 ≤ 20 μm, D90 ≤ 150 μm) | 200.0 |
| Lactose | 83.3 |
| Microcrystalline cellulose | 416.7 |
| Hydroxypropyl methylcellulose | 10.0 |
| Colloidal silica | 6.0 |
| Cross-linked sodium carboxymethyl cellulose (intragranular) | 15.0 |
| Magnesium stearate (intragranular) | 5.0 |
| Cross-linked sodium carboxymethyl cellulose (extragranular) | 45.0 |
| Magnesium stearate (extragranular) | 15.0 |

The preparation method was as described in Example 2, and the moisture content was further controlled within the range of 3.0% to 5.0%.

Example 4: Preparation of tablets of the compound of Formula I

Formulation composition of 1000 tablets of the compound of Formula I

| Name | Amount (g) |
|---|---|
| Compound of Formula I (amorphous form, D10 ≤ 8 μm, D50 ≤ 25 μm, D90 ≤ 160 μm) | 200.0 |
| Lactose | 133.3 |
| Microcrystalline cellulose | 66.7 |
| Hydroxypropyl methylcellulose | 2.0 |
| Colloidal silica | 2.0 |
| Cross-linked sodium carboxymethyl cellulose (intragranular) | 13.3 |
| Magnesium stearate (intragranular) | 1.3 |
| Cross-linked sodium carboxymethyl cellulose (extragranular) | 6.7 |
| Magnesium stearate (extragranular) | 0.7 |

The preparation method was as described in Example 2, and the moisture content was further controlled within the range of 3.0% to 5.0%.

Example 5: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 116.7 |
| Microcrystalline cellulose | 233.3 |
| Hydroxypropyl methylcellulose | 6.0 |
| Colloidal silica | 3.0 |

(continued)

| Name | Amount (g) |
|---|---|
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 6.0 |

The preparation method was as described in Example 2, and the moisture content was further controlled within the range of 3.0% to 5.0%.

Example 6: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 176.8 |
| Microcrystalline cellulose | 173.2 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

the preparation method was as described in Example 2, and the moisture content was further controlled within the range of 3.0% to 5.0%.

Example 7: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 206.4 |
| Microcrystalline cellulose | 142.6 |
| Hydroxypropyl methylcellulose | 6.0 |
| Colloidal silica | 3.0 |
| Cross-linked sodium carboxymethyl cellulose (intragranular) | 24.0 |
| Magnesium stearate (intragranular) | 6.0 |
| Cross-linked sodium carboxymethyl cellulose (extragranular) | 24.0 |
| Magnesium stearate (extragranular) | 6.0 |

The preparation method was as described in Example 2, and the moisture content was further controlled within the range of 3.0% to 5.0%.

Example 8: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 350.0 |
| Povidone | 6.0 |
| Talc | 3.0 |
| Hydroxypropyl cellulose (low-substituted) (intragranular) | 18.0 |
| Sodium stearyl fumarate (intragranular) | 4.8 |
| Hydroxypropyl cellulose (low-substituted) (extragranular) | 18.0 |
| Sodium stearyl fumarate (extragranular) | 4.8 |

Preparation method:

1) sizing the prescribed amounts of the compound of Formula I and lactose through a sieve, then mixing and screening the prescribed amounts of Povidone, hydroxypropyl cellulose (low-substituted) (intragranular) and talc, where the specification of the sieve is: Φ1.0 mm, and the frequency for the sizing is 8 Hz,

premixing 1 the screened materials in a mixer at a rotational speed of 10 rpm for 15 min to obtain a premix 1,

at the end of the premixing 1, adding sodium stearyl fumarate (intragranular) and premixing 2 at a rotational speed of 10 rpm for 5 min to obtain a premix 2.

2) putting the pre-mixture 2 into a dry granulation machine for dry granulation, where the feeding speed of the hopper is 5-10 rpm, the feeding speed of the screw is 10-20 rpm, the speed of the pressure wheel is 13-23 rpm, the hydraulic pressure is 60-80 bar, the gap of the pressure wheel is 1.0 mm, the sieve specification for the sizing 1 is 10 mesh, and the speed for the sizing 1 is 30-80 rpm, the sieve specification for the sizing 2 is 24 mesh, and the speed for the sizing 2 is 80-180 rpm. After sizing, dry granules are obtained. After the dry granulation is completed, samples are taken for the detection of drying weight loss, which shall not exceed 5.0%.

3) transferring the dry granules along with the calculated hydroxypropyl cellulose (low-substituted) (extragranular) to a mixer and pre-mix at a mixing speed of 10 rpm for 5 min to obtain a premix; then adding the calculated sodium stearyl fumarate (extragranular) and mixing with the premix in the mixer at a mixing speed of 10 rpm for 3 min to obtain a total mixture. After the total mixing is completed, samples are taken for the inspection of intermediate products.

4) after the content of the total mixed granules is inspected to be qualified, tableting under the following conditions: the die specification is 17 mm * 8.5 mm shallow concave punch, the tableting speed is 100-180 thousand tablets per hour, the main pressure of the tablet press is 8-11 kN, and the hardness is controlled within the range of 100 to 180 N. After the tableting is completed, samples are taken for the inspection of intermediate products.

5) weighing the film coating premix and purified water to formulate a coating solution, and coating using a high-efficiency coating machine under the following conditions: the inlet air temperature for coating is 40-66°C, the outlet air temperature is 35-55°C, the material temperature is 35-55°C, the liquid spraying speed is 0.1-0.5 kg/min, the atomization pressure is 1.0-5.0 bar, and the weight gain range of coating is 2.0-4.0%. After the coating is completed, samples are taken for the inspection of appearance, coating weight gain and drying weight loss, which shall not exceed 5.0%, and the moisture content is controlled within the range of 3.0% to 5.0% by adjusting the length of drying time.

Example 9: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 116.4 |
| Microcrystalline cellulose | 232.6 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

The preparation method was as described in Example 2, and the moisture content was further controlled within the range of 2.0% to 3.0%.

Example 10: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 116.4 |
| Microcrystalline cellulose | 232.6 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

The preparation method was as described in Example 2, and the moisture content was further controlled within the range of 1.0% to 2.0%.

Comparative Example 1: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 116.4 |
| Microcrystalline cellulose | 232.6 |
| Hydroxypropyl methylcellulose | 1.6 |
| Colloidal silica | 3.0 |

(continued)

| Name | Amount (g) |
|---|---|
| Cross-linked sodium carboxymethyl cellulose (intragranular) | 36.0 |
| Magnesium stearate (intragranular) | 24.0 |

1) sizing the prescribed amounts of the compound of Formula I and lactose through a sieve, then mixing and screening the prescribed amounts of hydroxypropyl methylcellulose, cross-linked sodium carboxymethyl cellulose and colloidal silica, and finally screening the prescribed amount of microcrystalline cellulose, where the specification of the sieve is: Φ1.0 mm, and the frequency for the sizing is 8 Hz,

premixing 1 the screened materials in a mixer at a rotational speed of 10 rpm for 15 min to obtain a premix 1,

at the end of the premixing 1, adding magnesium stearate and premixing 2 at a rotational speed of 10 rpm for 5 min to obtain a premix 2.

2) putting the pre-mixture 2 into a dry granulation machine for dry granulation, where the feeding speed of the hopper is 5-10 rpm, the feeding speed of the screw is 10-20 rpm, the speed of the pressure wheel is 13-23 rpm, the hydraulic pressure is 60-80 bar, the gap of the pressure wheel is 1.0 mm, the sieve specification for the sizing 1 is 10 mesh, and the speed for the sizing 1 is 30-80 rpm, the sieve specification for the sizing 2 is 24 mesh, and the speed for the sizing 2 is 80-180 rpm. After sizing, dry granules are obtained. After the dry granulation is completed, samples are taken for the detection of drying weight loss, which shall not exceed 5.0%.

3) after the content of the granules is inspected to be qualified, tableting under the following conditions: the die specification is 17 mm * 8.5 mm shallow concave punch, the tableting speed is 100-180 thousand tablets per hour, the main pressure of the tablet press is 8-11 kN, and the hardness is controlled within the range of 100-180 N. After the tableting is completed, samples are taken for the inspection of intermediate products.

4) weighing the film coating premix and purified water to formulate a coating solution, and coating using a high-efficiency coating machine under the following conditions: the inlet air temperature for coating is 40-66°C, the outlet air temperature is 35-55°C, the material temperature is 35-55°C, the liquid spraying speed is 0.1-0.5 kg/min, the atomization pressure is 1.0-5.0 bar, and the weight gain range of coating is 2.0-4.0%. After the coating is completed, samples are taken for the inspection of appearance, coating weight gain and drying weight loss, which shall not exceed 5.0%, and the moisture content is controlled within the range of 3.0% to 5.0% by adjusting the length of drying time.

Compared with Example 2, the amount of binder used in Comparative Example 1 is less, while the amount of lubricant is more. During the preparation process, the disintegrants and lubricants were not added intra-granularly or extra-granularly.

Comparative Example 2: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 85.7 |
| Microcrystalline cellulose | 514.3 |
| Hydroxypropyl methylcellulose | 6.0 |
| Colloidal silica | 3.0 |
| Cross-linked sodium carboxymethyl cellulose (intragranular) | 35.0 |
| Magnesium stearate (intragranular) | 12.0 |
| Cross-linked sodium carboxymethyl cellulose (extragranular) | 35.0 |

(continued)

| Name | Amount (g) |
|---|---|
| Magnesium stearate (extragranular) | 12.0 |

The preparation method was as described in Example 2, and the moisture content was further controlled within the range of 3.0% to 5.0%. In the formulation of Comparative Example 2, the amount of filler is more, and the proportion of microcrystalline cellulose in the mixed filler is relatively high. Meanwhile, the amounts of disintegrants and lubricants are also more.

Comparative Example 3: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 104.3 |
| Microcrystalline cellulose | 208.7 |
| Hydroxypropyl methylcellulose | 6.0 |
| Colloidal silica | 3.0 |
| Cross-linked sodium carboxymethyl cellulose (intragranular) | 18.0 |
| Cross-linked sodium carboxymethyl cellulose (extragranular) | 18.0 |
| Magnesium stearate | 6.0 |
| Tween | 36.0 |

In the Comparative Example 3, wet granulation was adopted. The preparation method was as follows:

1) preprocessing: pass the colloidal silica through a 35-mesh sieve and set it aside;

2) preparation of granulation solution: dissolve hydroxypropyl methylcellulose and Tween 80 in purified water and set it aside;

3) pre-mixing: weigh the prescribed amount of the compound of Formula I and the prescribed amounts of lactose, microcrystalline cellulose, and intragranular cross-linked sodium carboxymethyl cellulose, mix and pass them through a 60-mesh sieve to obtain a premix;

4) wet granulation: dropwise add the prepared granulation solution to the premix, and prepare wet granules that "form a ball when lightly held and disintegrate when lightly pressed" through manual wet granulation;

5) wet sizing: wet size the wet granules obtained by the wet granulation method using an 18-mesh sieve;

6) drying: dry the wet granules obtained after wet sizing in a precision blast drying oven, with the drying weight loss controlled at ≤3.0%;

7) dry sizing: dry size the dried granules using an 18-mesh sieve;

8) total mixing: calculate the extragranular cross-linked sodium carboxymethyl cellulose and colloidal silica based on the amount of dry granules, pass the extragranular cross-linked sodium carboxymethyl cellulose and colloidal silica through a 60-mesh sieve along with 3-fold amount of the extragranular cross-linked sodium carboxymethyl cellulose and colloidal silica dry granules and mix with the total dry granules; then add the calculated magnesium stearate for total mixing to obtain the total mixed granules;

9) tableting: compress the total mixed granules into tablets, with the moisture content further controlled within the

range of 3.0% to 5.0%.

Comparative Example 4: Preparation of tablets of the compound of Formula I

Formulation composition of 1000 tablets of the compound of Formula I

| Name | Amount (g) |
|---|---|
| Compound of Formula I (amorphous form, 10 ≤ D10 ≤ 12 μm, 28 ≤ D50 ≤ 35 μm, 165 ≤ D90 ≤ 180 μm) | 200.0 |
| Lactose | 116.4 |
| Microcrystalline cellulose | 232.6 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

The preparation method was as described in Example 2, and the moisture content was further controlled within the range of 3.0% to 5.0%. Compared with Example 2, the compound of Formula I in the formulation of Comparative Example 4 has a larger particle size.

Comparative Example 5: Preparation of tablets of the compound of Formula I

Formulation composition of 1000 tablets of the compound of Formula I

| Name | Amount (g) |
|---|---|
| Compound of Formula I (amorphous form, D10 ≤ 3 μm, D50 ≤ 15 μm, D90 ≤ 140 μm) | 200.0 |
| Lactose | 83.3 |
| Microcrystalline cellulose | 416.7 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

The preparation method was as described in Example 2, and the moisture content was controlled within the range of 6.0% to 8.0%. Compared with the formulation of Example 2, the moisture content in the formulation of Comparative Example 5 was controlled at greater than 5.0%.

Comparative Example 6: Preparation of tablets of the compound of Formula I

Formulation composition of 1000 tablets of the compound of Formula I

| Name | Amount (g) |
|---|---|
| Compound of Formula I (amorphous form+ crystal form I, D10 ≤ 3 μm, D50 ≤ 15 μm, D90 ≤ 140 μm) | 200.0 |

(continued)

| Name | Amount (g) |
|---|---|
| Lactose | 116.4 |
| Microcrystalline cellulose | 232.6 |
| Hydroxypropyl methylcellulose | 6.0 |
| Colloidal silica | 3.0 |
| Cross-linked sodium carboxymethyl cellulose (intragranular) | 18.0 |
| Magnesium stearate (intragranular) | 3.0 |
| Cross-linked sodium carboxymethyl cellulose (extragranular) | 18.0 |
| Magnesium stearate (extragranular) | 3.0 |

The preparation method was as described in Example 2, and the moisture content was controlled within the range of 3.0% to 5.0%. Compared with the formulation of Example 2, the active ingredient of the compound of Formula I in the formulation of Comparative Example 6 contains a certain proportion of the crystal form I of the compound of Formula I.

Example 11

The tablets of the compound of Formula I prepared in Examples 2-10 and Comparative Examples 1-6 were taken for quality investigation, and the results are as follows:

Table 2 Quality investigation of tablets of the compound of Formula I in Examples 2-10

| No. | Example 2 | Example 3 | Example 4 | Example 5 | Example 6 | Example 7 | Example 8 | Example 9 | Example 10 |
|---|---|---|---|---|---|---|---|---|---|
| Shape | The product is a film-coated tablet, appearing white or off-white after removing the coating | | | | | | | | |
| Sticking | No | No | No | No | No | No | No | No | No |
| Loosening or cracking | No | No | No | No | No | No | No | No | No |
| Content uniformity (A+2.2S ≤ 15) | Meet the require ments | Meet the require ments | Meet the require ments | Meet the require ments | Meet the require ments | Meet the require ments | Meet the require ments | Meet the require ments | Meet the require ments |
| Moisture (%) | 3.8 | 3.7 | 3.9 | 3.8 | 3.9 | 3.8 | 3.9 | 2.8 | 1.9 |

Table 3 Quality investigation of tablets of the compound of Formula I in Comparative Examples 1-6

| No. | Comparative Example 1 | Comparative Example 2 | Comparative Example 3 | Comparativ e Example 4 | Comparative Example 5 | Comparative Example 6 |
|---|---|---|---|---|---|---|
| Shape | The product is a film-coated tablet, appearing white or off-white after removing the coating | | | | | |
| Sticking | No | No | No | No | No | No |
| Loosening or cracking | Yes | No | No | No | No | No |
| Content uniformity (A+2.2S ≤ 15) | Meet the requirements | 15.6 | Meet the requirements | Meet the requiremen ts | Meet the requirements | Meet the requirements |

| Moisture (%) | 3.7 | 3.8 | 4.8 | 3.7 | 6.8 | 3.8 |
|---|---|---|---|---|---|---|

It can be seen from the above results that the tablets of the compound of Formula I prepared in Examples 2-10 and Comparative Examples 1-6 are all white or off-white tablets in shape after the coating is removed. However, for the tablets prepared from the formulation in Comparative Example 1, loosing and cracking occurred during the preparation process. After analysis, it can be known that the amount of binder used in this formulation is less and the amount of lubricant is more, and the mixing is inappropriate, resulting in uneven pressure distribution, and thus the occurrence of loosening and cracking situation. The content uniformity of the tablets prepared from the formulation of Comparative Example 2 exceeded the standard. After analysis, it can be known that the amounts of fillers, disintegrants and lubricants used in this formulation were too large, and the active ingredient and auxiliary materials could not be well mixed, thus failing to achieve the best uniformity effect. Examples 2-10 are the preferred schemes in this solution, including the types and proportions of auxiliary materials, all of which are the most optimal. For instance, a more appropriate proportion of lactose and microcrystalline cellulose are used as the mixed fillers, and disintegrants and lubricants are added both intra-granularly and extra-granularly, so that over-lubrication and loosening or cracking situations are less likely to occur during the mixing process, the obtained tablets have better compressibility, and the overall combination of the above conditions enables the tablets of the compound of Formula I to achieve the most optimal during the preparation process.

Example 12: Investigation of relevant substances in different formulations

Table 4: Investigation of relevant substances in tablets of the compound of Formula I

| | | Compound of Formula I, the active ingredient | Example 2 | Example 6 | Example 7 | Example 9 | Example 10 | Comparative Example 3 | Comparative Example 4 | Comparative Example 5 | Comparative Example 6 |
|---|---|---|---|---|---|---|---|---|---|---|---|
| Relevant substances (%) | RRT0.82 | 0.059 | 0.060 | 0.062 | 0.058 | 0.063 | 0.058 | 0.208 | 0.063 | 0.062 | 0.061 |
| | RRT0.95/0.9 6 | 0.241 | 0.265 | 0.255 | 0.259 | 0.256 | 0.251 | 0.447 | 0.288 | 0.263 | 0.266 |
| | Total impurities (%) | 4.800 | 4.921 | 4.951 | 4.924 | 4.952 | 4.944 | 5.400 | 4.955 | 4.966 | 4.935 |

It can be seen from the above results that, in the tablets prepared from the formulations of Examples 2, 6, 7, 9 and 10 and Comparative Examples 4, 5 and 6, there was no significant increase in the relevant substances (RRT0.82, RRT0.95/0.96), while in the tablets prepared from the formulation of Comparative Example 3, there was a significant increase in the relevant substances (RRT0.82, RRT0.95/0.96). The possible reasons are that the active ingredient of the compound of Formula I is sensitive to moisture and heat, and the addition of granulation solution during the wet granulation process as well as the drying process after granulation lead to the instability of the active ingredient of the compound of Formula I and the increase of impurities. The inventors subsequently conducted an investigation on the relevant substances in the tablets obtained from the formulations of Examples 3-5 and 8, and also found that there was no significant increase in the relevant substances (RRT0.82, RRT0.95/0.96). Examples 2-10 are the preferred schemes in this solution, in which dry granulation was adopted to fully reduce the impact of the relevant substances during the preparation process.

Example 13: Investigation on the distinguishing ability of dissolution medium

The distinguishing ability of pH 6.8 medium for different finished preparations was investigated by comparing the dissolution behavior differences between the finished preparations containing a certain proportion of the crystal form of the active ingredient and those containing the amorphous form of the active ingredient in the pH 6.8 medium.

Dissolution test: the composition of the compound of Formula I in the present disclosure was subjected to a dissolution test in 900 ml of phosphate buffer solution with a pH of 6.8, using the second dissolution method, the paddle method, at a speed of 75 rpm as stipulated in the Chinese Pharmacopoeia 2020 Edition, Part IV 0931. The results are shown in Table 5.

Table 5 Investigation on the distinguishing ability of dissolution medium

| Example /Comparative Example | Example 2 | Comparative Example 6 |
|---|---|---|
| Time (min) | Cumulative dissolution amount% ± SD | |
| 5 | 22.5±0.3 | 4.5±1.3 |
| 10 | 36.8±1.2 | 20.3±0.7 |
| 15 | 47.0±0.9 | 32.3±0.5 |
| 30 | 65.2±1.6 | 50.7±0.4 |
| 45 | 76.1±1.2 | 61.0±0.3 |
| 60 | 82.9±0.8 | 68.4±0.3 |
| 90 | 90.4±0.9 | 77.1±0.3 |
| 120 | 94.6±0.6 | 82.9±0.3 |

It can be seen from the above results and FIG. 3 that the tablets obtained from the formulation of Example 2 have a better dissolution in the phosphate buffer solution with a pH of 6.8, with a dissolution rate of over 43.0% within 15 min, over 60.0% within 30 min, over 70.0% within 60 min, and basically complete dissolution within 120 min; while in contrast, the formulation of Comparative Example 6 contains a certain proportion of crystal form I, and therefore, the dissolution rate within 60 min fails to reach 70.0%, and the dissolution within 120 min is incomplete. By determining the dissolution rates of the finished preparations containing a certain proportion of the crystal form I of the active ingredient and those containing the amorphous form of the active ingredient in the pH 6.8 medium, i.e. the phosphate buffer solution with a pH of 6.8, it was found that there was a significant difference in the dissolution rates, further indicating that the phosphate buffer solution with a pH of 6.8 has a certain distinguishing ability for the finished preparations.

Example 14: Investigation on the dissolution in multiple media

The dissolution test was conducted on the tablets of the compound of Formula I prepared in Examples 2, 6, 7, 9, 10, and Comparative Examples 2, 4 and 5. Samples were taken and measured at 5 min, 10 min, 15 min, 20 min, 30 min, 45 min, 60 min, 90 min and 120 min, and the average cumulative dissolution percentage was calculated. The investigation results of the dissolution rate are shown in Tables 6 and 7.

Table 6: Investigation on the dissolution rate of tablets of the compound of Formula I (pH 6.8, pH 1.0)

| Dis solu tionmed ium No. | Paddle method - 75 rpm - pH6.8 phosphate buffer solution - 900 ml (%, mean ± SD) | | | | | | | | Paddle method - 75 rpm - pH1.0 hydrochloric solution - 900 ml (%, mean ± SD)acid | | | | | | | |
|---|---|---|---|---|---|---|---|---|---|---|---|---|---|---|---|---|
| | Exa mpl e 2 | Exa mpl e 6 | Exa mpl e 7 | Exa mpl e 9 | Exa mpl e 10 | Co mpa rati ve Exa mpl e 2 | Co mpa rati ve Exa mpl e4 | Co mpa rati ve Exa mpl e 5 | Exa mpl e 2 | Exa mpl e 6 | Exa mpl e 7 | Exa mpl e 9 | Exa mpl e 10 | Co mpa rati ve Exa mpl e 2 | Co mpa rati ve Exa mpl e 4 | Co mpa rati ve Exa mpl e 5 |
| 5 min | 22. 5 ±0 .3 | 23. 5 ±0 .3 | 19. 2 ±0 .3 | 26. 5 ±0 .3 | 27. 2 ±0 .3 | 21. 2 ±0 .6 | 12. 0 ±0 .6 | 23. 4 ±0 .3 | 20. 4 ±0 .6 | 20. 6 ±0 .4 | 25. 5 ±0 .9 | 22. 6 ±0 .4 | 24. 2 ±0 .9 | 22. 5 ±0 .7 | 12. 3 ±1 .2 | 18. 1 ±0 .6 |
| 10 min | 36. 8 ±1 .2 | 35. 0 ±0 .3 | 38. 6 ±0 .5 | 38. 8 ±0 .3 | 39. 6 ±0 .5 | 36. 0 ±0 .7 | 26. 0 ±1 .3 | 36. 1 ±0 .3 | 37. 6 ±0 .5 | 36. 8 ±0 .4 | 41. 7 ±0 .7 | 38. 8 ±0 .4 | 41. 7 ±0 .7 | 37. 9 ±0 .9 | 27. 2 ±1 .2 | 34. 2 ±0 .5 |
| 15 min | 47. 0 ±0 .9 | 45. 0 ±0 .4 | 50. 9 ±0 .5 | 50. 2 ±0 .4 | 53. 9 ±0 .5 | 46. 1 ±0 .8 | 36. 6 ±0 .8 | 48. 2 ±0 .4 | 48. 9 ±0 .5 | 48. 0 ±0 .4 | 51. 9 ±0 .6 | 48. 1 ±0 .4 | 52. 2 ±0 .6 | 48. 6 ±0 .9 | 37. 7 ±1 .2 | 45. 2 ±0 .3 |
| 30 min | 65. 2 ±1 .6 | 62. 8 ±0 .5 | 70. 5 ±0 .5 | 69. 8 ±0 .5 | 70. 5 ±0 .5 | 54. 2 ±1 .0 | 45. 0 ±0 .8 | 66. 3 ±0 .5 | 69. 0 ±0 .5 | 67. 4 ±0 .6 | 69. 9 ±0 .6 | 69. 4 ±0 .6 | 70. 9 ±0 .6 | 58. 0 ±1 .0 | 46. 5 ±0 .9 | 65. 1 ±0 .5 |
| 45 min | 76. 1 ±1 .2 | 73. 1 ±0 .7 | 80. 4 ±0 .7 | 79. 1 ±0 .7 | 80. 4 ±0 .7 | 65. 4 ±1 .1 | 55. 3 ±0 .8 | 76. 2 ±0 .7 | 80. 0 ±0 .5 | 77. 9 ±0 .7 | 80. 0 ±0 .5 | 82. 1 ±0 .7 | 83. 0 ±0 .5 | 69. 0 ±0 .5 | 57. 2 ±0 .9 | 80. 0 ±0 .5 |
| 60 min | 82. 9 ±0 .8 | 80. 0 ±0 .6 | 85. 9 ±0 .8 | 86. 0 ±0 .6 | 85. 9 ±0 .8 | 72. 5 ±0 .9 | 61. 9 ±0 .8 | 83. 0 ±0 .5 | 86. 7 ±0 .6 | 84. 1 ±0 .6 | 86. 0 ±0 .5 | 87. 1 ±0 .6 | 88. 1 ±0 .5 | 75. 7 ±0 .4 | 63. 7 ±0 .7 | 84. 1 ±0 .6 |
| 90 min | 90. 4 ±0 .9 | 88. 1 ±0 .6 | 91. 8 ±0 .7 | 92. 1 ±0 .6 | 91. 8 ±0 .7 | 80. 4 ±0 .7 | 69. 3 ±0 .7 | 90. 1 ±0 .6 | 93. 8 ±0 .5 | 91. 0 ±0 .5 | 92. 4 ±0 .8 | 93. 0 ±0 .5 | 93. 8 ±0 .8 | 82. **7** **±0** .3 | 71. 2 ±0 .6 | 91. 8 ±0 .5 |
| 120 min | 94. 6 ±0 .6 | 92. 6 ±0 .6 | 94. 3 ±0. 5 | 93. 6 ±0 .6 | 95. 3 ±0. 5 | 83. 7 ±0 .7 | 73. 2 ±0 .8 | 93. 2 ±0 .6 | 97. 3 ±0 .4 | 94. 5 ±0 .7 | 95. 6 ±0 .9 | 96. 2 ±0 .7 | 96. 1 ±0 .9 | 86. 2 ±0 .4 | 74. 8 ±0 .3 | 94. 1 ±0 .4 |

Table 7: Investigation on the dissolution rate of tablets of the compound of Formula I (pH4.5, water)

| Dissolution medium | Paddle method - 75 rpm - pH4.5 acetate buffer ml (%, mean ± SD)- 900 | | | | | | | | Paddle method - 75 rpm - water - 900 ml (%, mean SD)± | | | | | | | |
|---|---|---|---|---|---|---|---|---|---|---|---|---|---|---|---|---|
| No. | Example 2 | Example 6 | Example 7 | Example 9 | Example 10 | Comparative Example 2 | Comparative Example 4 | Comparative Example 5 | Example 2 | Example 6 | Example 7 | Example 9 | Example 10 | Comparative Example 2 | Comparative Example 4 | Comparative Example 5 |
| 5 min | 23.8 ±0. 6 | 25.2 ±0. 7 | 26.5 ±1. 6 | 27.6 ±0. 7 | 28.5 ±1. 6 | 26.3 ±0. 3 | 14.8 ±0. 2 | 23.1 ±0. 6 | 22.0 ±0. 7 | 25.8 ±0. 8 | 22.5 ±0. 3 | 27.8 ±0. 8 | 28.5 ±0. 3 | 26.1 ±0. 6 | 17.0 ±0. 3 | 21.5 ±0. 7 |
| 10 min | 41.5 ±0. 3 | 39.9 ±0. 9 | 41.9 ±1. 4 | 44.9 ±0. 9 | 45.1 ±1. 4 | 42.4 ±0. 3 | 20.1 ±0. 3 | 41.1 ±0. 3 | 40.3 ±0. 4 | 41.2 ±0. 8 | 46.8 ±1. 2 | 43.1 ±0. 8 | 47.8 ±1. 1 | 42.2 ±0. 6 | 21.8 ±0. 3 | 40.3 ±0. 4 |
| 15 min | 52.9 ±0. 3 | 50.4 ±0. 9 | 52.0 ±1. 4 | 54.6 ±0. 9 | 55.0 ±1. 4 | 53.6 ±0. 4 | 30.5 ±0. 5 | 50.5 ±0. 3 | 51.5 ±0. 4 | 52.2 ±0. 6 | 57.0 ±0. 9 | 54.2 ±0. 6 | 58.0 ±0. 8 | 53.5 ±0. 4 | 31.6 ±0. 4 | 49.8 ±0. 4 |
| 30 min | 73.0 ±0. 4 | 68.6 ±0. 9 | 69.5 ±1. 6 | 76.6 ±0. 5 | 77.5 ±1. 6 | 63.1 ±0. 4 | 49.0 ±0. 6 | 71.2 ±0. 4 | 71.1 ±0. 7 | 71.6 ±0. 4 | 75.2 ±1. 6 | 73.6 ±0. 4 | 77.2 ±0. 9 | 62.9 ±0. 3 | 49.7 ±0. 4 | 70.1 ±0. 7 |
| 45 min | 83.6 ±0. 5 | 78.5 ±0. 9 | 79.6 ±1. 3 | 86.5 ±0. 6 | 87.6 ±1. 3 | 73.1 ±0. 5 | 59.3 ±0. 7 | 81.6 ±0. 5 | 81.7 ±0. 7 | 81.8 ±0. 4 | 86.1 ±1. 2 | 84.8 ±0. 4 | 88.1 ±1. 1 | 72.8 ±0. 5 | 59.9 ±0. 4 | 81.2 ±0. 7 |
| 60 min | 89.5 ±0. 6 | 84.7 ±1. 0 | 85.5 ±1. 4 | 91.7 ±1. 0 | 92.5 ±1. 4 | 78.8 ±0. 5 | 65.4 ±0. 7 | 87.2 ±0. 6 | 87.9 ±0. 9 | 87.7 ±0. 3 | 89.0 ±0. 8 | 89.2 ±0. 3 | 91.0 ±0. 6 | 78.8 ±0. 5 | 66.0 ±0. 5 | 86.1 ±0. 9 |
| 90 min | 95.6 ±0. 7 | 91.9 ±0. 8 | 92.1 ±1. 4 | 96.9 ±0. 8 | 97.1 ±1. 4 | 84.8 ±0. 6 | 72.1 ±0. 6 | 94.2 ±0. 7 | 94.8 ±0. 9 | 94.3 ±0. 9 | 95.4 ±0. 9 | 96.3 ±0. 8 | 97.4 ±0. 9 | 85.1 ±0. 5 | 72.9 ±0. 4 | 94.1 ±0. 9 |
| 120 min | 98.6 ±0. 6 | 95.4 ±0. 5 | 95.5 ±1. 3 | 98.4 ±0. 5 | 98.5 ±1. 0 | 87.2 ±0. 6 | 75.6 ±0. 5 | 97.8 ±0. 6 | 97.9 ±0. 9 | 96.9 ±0. 8 | 97.6 ±0. 6 | 97.9 ±0. 8 | 98.6 ±0. 3 | 87.5 ±0. 9 | 76.4 ±0. 5 | 95.1 ±0. 6 |

It can be seen from the above results that the tablets obtained from the formulations of Examples 2, 6, 7, 9 and 10 have a better dissolution in the phosphate buffer solution with a pH of 6.8, with a dissolution rate of over 43.0% within 15 min, over 60.0% within 30 min, and over 70.0% within 60 min, and further have a better dissolution in multiple media (pH 1.0, pH 4.5, pH 6.8 and purified water), basically complete dissolution within 120 min, showing excellent dissolution.

The tablets obtained from the formulation of Comparative Example 2 maintained normal dissolution in multiple media (pH 1.0, pH 4.5, pH 6.8 and purified water) before 30 min, and the dissolution time was prolonged after 30 min, and the tablets could not be completely dissolved within 120 min. After analysis, it can be known that in this formulation, the amount of microcrystalline cellulose used is too large, and the "bottom attaching" phenomenon gradually occurs during the later stage of dissolution, with microcrystals accumulating at the bottom of the dissolution cup, resulting in incomplete drug release and adversely affecting drug dissolution.

The tablets obtained from the formulation of Comparative Example 4 have a relatively long dissolution time in multiple media (pH 1.0, pH 4.5, pH 6.8 and purified water), and cannot be completely dissolved within 120 min. After analysis, it can be known that in the formulation of Comparative Example 4, the particle size of the active ingredient of the compound of Formula I is relatively large, and the mixing uniformity of the preparations is poor, which affects the subsequent release.

In the formulations of Examples 2, 6, 7, 9 and 10, the particle size of the active ingredient of the compound of Formula I is appropriate, and the type and amount of the formulations are within the optimal range. Therefore, the dissolution is good in multiple media (pH 1.0, pH 4.5, pH 6.8 and purified water).

Example 15: Stability study

The samples for the stability test (the tablets of the compound of Formula I prepared in Examples 2, 6, 7, 9 and 10 and Comparative Example 5) were packaged with "polyvinyl chloride/polyvinylidene chloride solid pharmaceutical composite hard plates + pharmaceutical aluminum foil (plain aluminum)" as the inner packaging and "polyester/aluminum/polyethylene pharmaceutical packaging composite film" as the outer packaging.

The stability in an accelerated test was investigated under the proposed packaging conditions. The samples were placed under the conditions of 40°C±2°C and 75%±5%RH for 3 months, and no significant changes occurred in each test item.

Table 8: Data sheet of stability in accelerated test

| Test item | Standard | Example / Comparative Example | 0M | 1M | 2M | 3M |
|---|---|---|---|---|---|---|
| Characteristics | The product is a film-coated tablet, appearing white or off-white after removing the coating | Example 2 | a film-coated tablet, appearing white after removing the coating | a film-coated tablet, appearing white after removing the coating | a film-coated tablet, appearing white after removing the coating | a film-coated tablet, appearing white after removing the coating |
| | | Example 6 | | | | |
| | | Example 7 | | | | |
| | | Example 9 | | | | |
| | | Example 10 | | | | |
| | | Comparative Example 5 | | | | |
| Content | should be 90.0%-110.0% of the labeled amount | Example 2 | 99.5% | 100.4% | 101.1% | 98.9% |
| | | Example 6 | 99.8% | 100.1% | 100.1% | 98.5% |
| | | Example 7 | 99.4% | 101.4% | 99.7% | 98.4% |
| | | Example 9 | 99.9% | 100.4% | 100.5% | 98.9% |
| | | Example 10 | 99.8% | 101.5% | 99.9% | 98.8% |
| | | Comparative Example 5 | 99.7% | 100.2% | 100.8% | 99.0% |

(continued)

| Test item | Standard | Example / Comparative Example | 0M | 1M | 2M | 3M |
|---|---|---|---|---|---|---|
| Isomers | ≤ 4.0% | Example 2 | 0.31% | 0.74% | 1.0% | 1.0% |
| | | Example 6 | 0.32% | 0.75% | 1.0% | 1.1% |
| | | Example 7 | 0.35% | 0.84% | 1.3% | 1.4% |
| | | Example 9 | 0.34% | 0.76% | 1.0% | 1.2% |
| | | Example 10 | 0.37% | 0.89% | 1.2% | 1.3% |
| | | Comparative Example 5 | 0.39% | 0.91% | 1.1% | 1.3% |
| Dissolution rate | should not less than 75% of the labeled amount | Example 2 | 81.7%-84.1% | 74.2%-75.5% | 75.1%-76.4% | 73.5%-75.1% |
| | | Example 6 | 80.7%-82.2% | 76.2%-77.7% | 75.0%-75.6% | 74.5%-76.4% |
| | | Example 7 | 81.0%-83.1% | 74.8%-75.7% | 74.0%-75.6% | 73.5%-75.1% |
| | | Example 9 | 84.7%-87.2% | 78.2%-79.7% | 77.2%-78.0% | 76.5%-77.3% |
| | | Example 10 | 85.0%-88.8% | 79.5%-80.1% | 78.8%-79.2% | 78.0%-78.9% |
| | | Comparative Example 5 | 81.7%-82.8% | 73.2%-74.9% | 65.2%-67.4% | 52.8%-54.3% |
| Impurities | **total impurities** ≤ 5.0% | Example 2 | 0.95% | 1.0% | 1.1% | 1.1% |
| | | Example 6 | 0.98% | 1.2% | 1.1% | 1.2% |
| | | Example 7 | 0.94% | 1.0% | 1.1% | 1.2% |
| | | Example 9 | 0.98% | 1.2% | 1.1% | 1.2% |
| | | Example 10 | 0.94% | 1.0% | 1.1% | 1.2% |
| | | Comparative Example 5 | 1.05% | 1.1% | 1.2% | 1.3% |
| Moisture | ≤ 5.0% | Example 2 | 4.53% | 4.19% | 4.72% | 4.06% |
| | | Example 6 | 4.63% | 4.21% | 4.93% | 4.09% |
| | | Example 7 | 4.75% | 4.22% | 4.83% | 4.14% |
| | | Example 9 | 2.82% | 2.53% | 2.91% | 2.33% |
| | | Example 10 | 1.92% | 1.65% | 2.25% | 1.81% |
| | | Comparative Example 5 | 6.84% | 6.73% | 7.33% | 7.22% |
| PXRD | should be consistent with the spectrogram on Day 0 | Example 2 | reported value | consistent with Day 0 | consistent with Day 0 | consistent with Day 0 |
| | | Example 6 | | | | |
| | | Example 7 | | | | |
| | | Example 9 | | | | |
| | | Example 10 | | | | |
| | | Comparative Example 5 | | | | |

It can be seen from the above results that after the tablets obtained from the formulations of Examples 2, 6, 7, 9 and 10 were investigated under accelerated test conditions (40°C±2°C, 75%±5%RH) for 3 months, no significant changes occurred in the test items of characteristics, content, isomers, dissolution rate, impurities, moisture and crystal form, and the moisture content of the tablets obtained from the formulations of Examples 2, 6, 7, 9 and 10 was controlled not exceeding 5.0%, and the dissolution was not affected. At the same time, it can be seen that under long-term storage, the lower the moisture content, the higher the dissolution (moisture content: Example 2 > Example 9 > Example 10, dissolution: Example 2 < Example 9 < Example 10).
As shown in FIG. 4, the dissolution rate of the tablets obtained from the formulation of Comparative Example 5 gradually decreased over time. After analysis, it can be known that when the moisture content of the tablets was too high, a "caking" phenomenon gradually occurred inside during storage, which in turn affected the dissolution of the preparations and led to a decline in the dissolution rate.
In conclusion, the composition of the compound of Formula I prepared in this solution has excellent quality of preparations, with no problems such as sticking, loosening or cracking, and has good dissolution and stability even after long-term storage, which are in line with the medicinal effects. Moreover, the preparation process is simple to operate and suitable for industrial production.

Example 16

The tablets used in this Example are prepared according to the formulation and preparation method in Example 2.
To verify the therapeutic effect of the pharmaceutical composition of the present disclosure, a clinical study was conducted using the tablets of the compound of Formula I prepared in Example 2 of the present disclosure as the test drug. The study process and results are as follows:

I. Related standards

1.1 Diagnostic criteria

1.1.1 Diagnosis of western medical diseases

According to the "Diagnosis and Treatment Protocol for COVID-19 (Trial Version 10)", if there is an epidemiological history and clinical manifestations, it meets the etiological evidence (positive nucleic acid test or positive antigen test for COVID-19).
According to the "Diagnosis and Treatment Protocol for COVID-19 (Trial Version 10)", the disease is classified into mild, common, severe and critical types.

1.2 Inclusion criteria

(1) Those with positive nucleic acid or antigen tests for COVID-19;

(2) According to the classification standards of the "Diagnosis and Treatment Protocol for COVID-19 (Trial Version 10)", those classified as mild cases.

(3) Those aged between 18-65 years old (including the boundary values);

(4) Those who have signed the informed consent form.

Exclusion criteria:

(1) Patients with severe underlying diseases or acute exacerbations of chronic diseases, such as acute heart failure;

(2) Female subjects during pregnancy, breastfeeding or lactation period;

(3) Patients suffering from diseases that affect the duration of symptoms, such as bronchopneumonia, chronic lung abscess, chronic cough for more than 8 weeks, and chronic pharyngitis with cough.

1.3 Withdrawal and dropout of cases

The withdrawal and dropout of cases refer to those where patients, after enrollment, are either unwilling to continue participating in clinical trials or fail to complete the entire course of treatment as required during the research process.

Handling of withdrawal and dropout cases: Researchers should contact the subjects through home visits, phone calls, letters, etc., record the time of the last treatment, and complete all possible assessment items, understand the reasons for withdrawal and truthfully record them in the case report form, and keep the observation data of all withdrawal and dropout cases.

II. Case source: The First Affiliated Hospital of Guangzhou Medical University and many other clinical research centers across the country.

III. Intervention and grouping:

All patients enrolled in the group of the compound of Formula I were given tablets of the compound of Formula I prepared in Example 2, 3 times a day, 2 tablets each time, with each tablet having a specification of 200 mg.

All patients were diagnosed and treated in accordance with the "Diagnosis and Treatment Protocol for COVID-19 (Trial Version 9)" after enrollment, ensuring the basic treatment for the patients, but no antiviral treatment was given.

IV. Statistical methods:

Normality tests were conducted on the measurement data. Data conforming to a normal distribution were represented as mean ± standard deviation, while those not conforming to the normal distribution were described by median and quartile. The therapeutic effects were compared successively. Paired t-tests were applied to normally distributed data, while non-parametric tests were applied to non-normally distributed data. Counting data were analyzed by the chi-square test.

V. Test results

Table 9: Main statistical analysis and sensitivity analysis (ITT) of time to sustained clinical recovery (based on 11 symptoms)

| Items | Placebo group (N=679) | Group of the compound of Formula I (N=680) | Total (N=1359) |
|---|---|---|---|
| Subject cases | 679 | 680 | 1359 |
| Number of events (%) | 538 (79.2%) | 569 (83.7%) | 1107 (81.5%) |
| Number of missing (%) | 141 (20.8%) | 111 (16.3%) | 252 (18.5%) |
| Use of prohibited drugs [1] | 11 (1.6%) | 5 (0.7%) | 16 (1.2%) |
| Switching to other treatments due to lack of therapeutic effects | 0 | 0 | 0 |
| Disease deterioration, conversion to severe or critical illness | 0 | 0 | 0 |
| Death caused by COVID-19 | 0 | 0 | 0 |
| Death caused by other reasons | 0 | 0 | 0 |
| Withdraw from the trial as requested by the subjects | 11 (1.6%) | 7 (1.0%) | 18 (1.3%) |
| Other reasons | 119 (17.5%) | 99 (14.6%) | 218 (16.0%) |
| Time to sustained clinical recovery (h) | | | |
| P25 (95%CI) | 219.00 (204.83, 238.10) | 188.95 (173.17, 211.62) | 208.40 (193.45, 217.20) |
| Median time (95%CI) | 271.33 (264.72, 294.20) | 251.02 (246.48, 268.13) | 265.97 (261.83, 273.28) |
| P75 (95%CI) | 529.63 (463.75, 560.58) | 428.68 (384.33, 466.82) | 464.02 (432.28, 509.73) |
| Stratified log-rank test [2] (Group of the compound of Formula I vs. Placebo group) | | | |
| P-value | | 0.002 | |

(continued)

| Items | Placebo group (N=679) | Group of the compound of Formula I (N=680) | Total (N=1359) |
|---|---|---|---|
| P-value* | | 0.003 | |
| * Sensitivity analysis using the planned stratification factors. | | | |

It can be known from the results in Table 9 that based on the ITT analysis, among the 1359 subjects, a total of 1107 cases (81.5%) achieved clinical recovery, including 538 cases (79.2%) in the placebo group and 569 cases (83.7%) in the group of the compound of Formula I. The median time to sustained clinical recovery for the 11 symptoms in the group of the compound of Formula I and the placebo group was 251.02 h (95%CI: 246.48, 268.13) and 271.33 h (95%CI: 264.72, 294.20), respectively. The actual baseline clinical classification of the subjects and whether they were in the high-risk population of severe/critical cases were regarded as the stratification factors for adjustment. The stratified log-rank test was used to compare the difference in the median time to sustained clinical recovery between the two groups, and the difference was statistically significant (P=0.002). The time to sustained clinical recovery for the 11 symptoms in the group of the compound of Formula I was 20.31 hours shorter than that in the placebo group. It can be considered that the group of the compound of Formula I was superior to the placebo group in shortening the time to sustained clinical recovery for the 11 symptoms. It can be known that the composition prepared in the present disclosure can achieve an ideal clinical therapeutic effect when the dissolution behavior conditions of the present invention are met.

In conclusion, the composition of the compound of Formula I prepared in this solution has excellent quality of preparations, with no problems such as sticking, loosening or cracking, and has good dissolution and stability, which are in line with the medicinal effects. Moreover, the preparation process is simple to operate and suitable for industrial production.

The above examples are the preferred embodiments of the present disclosure. However, the embodiments of the present disclosure are not limited by the above examples. Any other changes, modifications, substitutions, combinations, and simplifications made without deviating from the spirit and principle of the present disclosure shall be equivalent replacements and are all included within the protection scope of the present disclosure.

## Claims

1. A pharmaceutical composition, **characterized by** comprising a compound of Formula I or a pharmaceutically acceptable salt thereof and an auxiliary material; the auxiliary material comprises one or more of a filler, a binder, a glidant, a disintegrant and a lubricant; the filler is one or more of lactose, anhydrous lactose, microcrystalline cellulose, mannitol or starch; the binder is one or more of hydroxypropyl methylcellulose, hydroxypropyl cellulose, Povidone, hydroxyethyl cellulose, sodium carboxymethyl cellulose or polyvinyl alcohol; the glidant is one or more of colloidal silica, talc or micronized silica gel; the disintegrant is one or more of low-substituted hydroxypropyl cellulose, cross-linked sodium carboxymethyl cellulose, sodium carboxymethyl starch or cross-linked Povidone; the lubricant is one or more of magnesium stearate, calcium stearate or sodium stearyl fumarate; a mass ratio of the compound of Formula I to the filler is 1:1.0-2.5, a mass ratio of the compound of Formula I to the binder is 1:0.01-0.05, a mass ratio of the compound of Formula I to the glidant is 1:0.01-0.03, a mass ratio of the compound of Formula I to the disintegrant is 1:0.10-0.30, and a mass ratio of the compound of Formula I to the lubricant is 1:0.01-0.10;

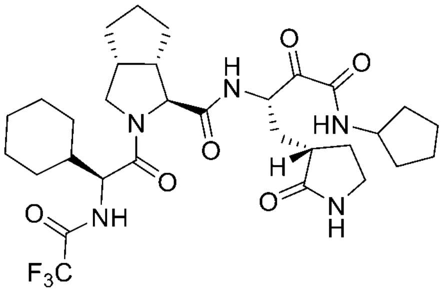

I.

2. A pharmaceutical composition, **characterized by** comprising a compound of Formula I or a pharmaceutically

acceptable salt thereof and an auxiliary material; the auxiliary material comprises one or more of a filler, a binder, a glidant, a disintegrant and a lubricant; the filler is one or more of lactose, anhydrous lactose, microcrystalline cellulose, mannitol or starch; the binder is one or more of hydroxypropyl methylcellulose, hydroxypropyl cellulose, Povidone, hydroxyethyl cellulose, sodium carboxymethyl cellulose or polyvinyl alcohol; the glidant is one or more of colloidal silica, talc or micronized silica gel; the disintegrant is one or more of low-substituted hydroxypropyl cellulose, cross-linked sodium carboxymethyl cellulose, sodium carboxymethyl starch or cross-linked Povidone; the lubricant is one or more of magnesium stearate, calcium stearate or sodium stearyl fumarate; a mass ratio of the compound of Formula I to the filler is 1:1.0-2.5, a mass ratio of the compound of Formula I to the binder is 1:0.01-0.05, a mass ratio of the compound of Formula I to the glidant is 1:0.01-0.03, a mass ratio of the compound of Formula I to the disintegrant is 1:0.10-0.30, a mass ratio of the compound of Formula I to the lubricant is 1:0.01-0.10; and the pharmaceutical composition has a moisture content not exceeding 5.0%;

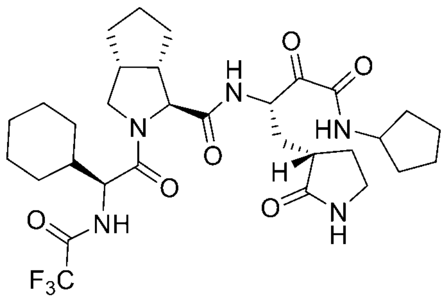

I.

3. A pharmaceutical composition, **characterized by** comprising a compound of Formula I or a pharmaceutically acceptable salt thereof and an auxiliary material; the auxiliary material comprises one or more of a filler, a binder, a glidant, a disintegrant and a lubricant; the filler is one or more of lactose, anhydrous lactose, microcrystalline cellulose, mannitol or starch; the binder is one or more of hydroxypropyl methylcellulose, hydroxypropyl cellulose, Povidone, hydroxyethyl cellulose, sodium carboxymethyl cellulose or polyvinyl alcohol; the glidant is one or more of colloidal silica, talc or micronized silica gel; the disintegrant is one or more of low-substituted hydroxypropyl cellulose, cross-linked sodium carboxymethyl cellulose, sodium carboxymethyl starch or cross-linked Povidone; the lubricant is one or more of magnesium stearate, calcium stearate or sodium stearyl fumarate; the pharmaceutical composition exhibits a dissolution rate of 70.0% or more at 60 min in a phosphate buffer solution with a pH of 6.8 as a dissolution medium;

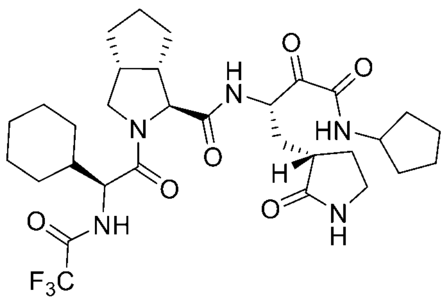

I.

4. The pharmaceutical composition according to any one of claims 1 to 3, wherein the filler is a mixture of lactose and microcrystalline cellulose, the binder is hydroxypropyl methylcellulose, the glidant is colloidal silica, the disintegrant is cross-linked sodium carboxymethyl cellulose, and the lubricant is magnesium stearate.

5. The pharmaceutical composition according to claim 3, wherein a mass ratio of the compound of Formula I to the filler is 1:1.0-2.5, a mass ratio of the compound of Formula I to the binder is 1:0.01-0.05, a mass ratio of the compound of Formula I to the glidant is 1:0.01-0.03, a mass ratio of the compound of Formula I to the disintegrant is 1:0.10-0.30, and a mass ratio of the compound of Formula I to the lubricant is 1:0.01-0.10.

6. The pharmaceutical composition according to any one of claims 1 to 3, wherein the mass ratio of the compound of

Formula I to the filler is 1:1.5-2.0, the mass ratio of the compound of Formula I to the binder is 1:0.02-0.04, the mass ratio of the compound of Formula I to the glidant is 1:0.012-0.020, the mass ratio of the compound of Formula I to the disintegrant is 1:0.15-0.25, the mass ratio of the compound of Formula I to the lubricant is 1:0.02-0.06; preferably, the mass ratio of the compound of Formula I to the lubricant is 1:0.02-0.05; preferably, the mass ratio of the compound of Formula I to the filler is 1:1.75, the mass ratio of the compound of Formula I to the binder is 1:0.03, the mass ratio of the compound of Formula I to the glidant is 1:0.015, the mass ratio of the compound of Formula I to the disintegrant is 1:0.18 or 1:0.24, and the mass ratio of the compound of Formula I to the lubricant is 1:0.03 or 1:0.06.

**7.** The pharmaceutical composition according to claim 2, wherein the pharmaceutical composition has a moisture content of 1.0% to 5.0%, preferably 3.0% to 5.0%.

**8.** The pharmaceutical composition according to claim 3, wherein the pharmaceutical composition exhibits a dissolution rate of 43.0% or more at 15 min in a phosphate buffer solution with a pH of 6.8 as a dissolution medium.

**9.** The pharmaceutical composition according to claim 3, wherein the pharmaceutical composition exhibits a dissolution rate of 60.0% or more at 30 min in a phosphate buffer solution with a pH of 6.8 as a dissolution medium.

**10.** The pharmaceutical composition according to any one of claims 1 to 3, wherein the disintegrant is added both intra-granularly and extra-granularly during a preparation, and the lubricant is added both intra-granularly and extra-granularly during a preparation.

**11.** The pharmaceutical composition according to claim 10, wherein when the disintegrant is added both intra-granularly and extra-granularly during the preparation, a mass ratio of the intragranular disintegrant to the extragranular disintegrant is 1:0.5-3; preferably, the mass ratio of the intragranular disintegrant to the extragranular disintegrant is 1:0.8-2; more preferably, the mass ratio of the intragranular disintegrant to the extragranular disintegrant is 1:1.

**12.** The pharmaceutical composition according to claim 10, wherein when the lubricant is added both intra-granularly and extra-granularly during the preparation, a mass ratio of the intragranular lubricant to the extragranular lubricant is 1:0.5-3; preferably, the mass ratio of the intragranular lubricant to the extragranular lubricant is 1:0.8-2; more preferably, the mass ratio of the intragranular lubricant to the extragranular lubricant is 1:1.

**13.** The pharmaceutical composition according to claim 4, wherein a mass ratio of lactose to microcrystalline cellulose is 1:0.5-5; preferably, the mass ratio of lactose to microcrystalline cellulose is 1:0.6-3; more preferably, the mass ratio of lactose to microcrystalline cellulose is 1:1-3; most preferably, the mass ratio of lactose to microcrystalline cellulose is 1:0.69, 1:0.98 or 1:2.

**14.** The pharmaceutical composition according to any one of claims 1 to 3, wherein the compound of Formula I has a particle size of D10 $\leq$ 8 $\mu$m, D50 $\leq$ 25 $\mu$m, D90 $\leq$ 160 $\mu$m; preferably, the compound of Formula I has a particle size of D10 $\leq$ 5 $\mu$m, D50 $\leq$ 20 $\mu$m, D90 $\leq$ 150 $\mu$m; more preferably, the compound of Formula I has a particle size of D10 $\leq$ 3 $\mu$m, D50 $\leq$ 15 $\mu$m, D90 $\leq$ 140 $\mu$m.

**15.** The pharmaceutical composition according to any one of claims 1 to 3, wherein the compound of Formula I is in amorphous form.

**16.** A pharmaceutical composition, **characterized by** comprising the following components:

| name | amount, mg/tablet |
|---|---|
| 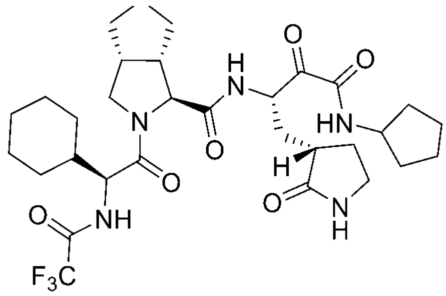 compound of Formula I | 200.0 |
| lactose | 116.4 |
| microcrystalline cellulose | 232.6 |
| hydroxypropyl methylcellulose | 6.0 |
| colloidal silica | 3.0 |
| cross-linked sodium carboxymethyl cellulose - intragranular | 18.0 |
| magnesium stearate - intragranular | 3.00 |
| cross-linked sodium carboxymethyl cellulose - extragranular | 18.0 |
| magnesium stearate - extragranular | 3.0 |

; the compound of Formula I has a particle size of D10 ≤ 3 μm, D50 ≤ 15 μm, D90 ≤ 140 μm; and the compound of Formula I is in amorphous form.

17. A pharmaceutical composition, **characterized by** comprising the following components:

| name | amount, mg/tablet |
|---|---|
| 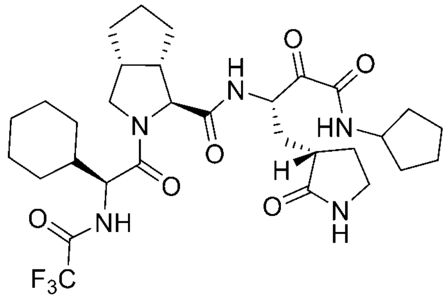 compound of Formula I | 200.0 |
| lactose | 176.8 |
| microcrystalline cellulose | 173.2 |
| hydroxypropyl methylcellulose | 6.0 |
| colloidal silica | 3.0 |
| cross-linked sodium carboxymethyl cellulose - intragranular | 18.0 |
| magnesium stearate - intragranular | 3.0 |
| cross-linked sodium carboxymethyl cellulose - extragranular | 18.0 |
| magnesium stearate - extragranular | 3.0 |

; the compound of Formula I has a particle size of D10 ≤ 3 μm, D50 ≤ 15 μm, D90 ≤ 140 μm; and the compound

of Formula I is in amorphous form.

**18.** A pharmaceutical composition, **characterized by** comprising the following components:

| name | amount, mg/tablet |
|---|---|
| 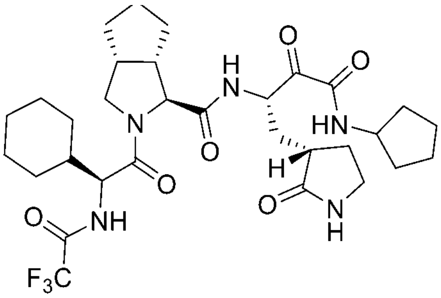 compound of Formula I | 200.0 |
| lactose | 206.4 |
| microcrystalline cellulose | 142.6 |
| hydroxypropyl methylcellulose | 6.0 |
| colloidal silica | 3.0 |
| cross-linked sodium carboxymethyl cellulose - intragranular | 24.0 |
| magnesium stearate - intragranular | 6.0 |
| cross-linked sodium carboxymethyl cellulose - extragranular | 24.0 |
| magnesium stearate - extragranular | 6.0 |

; the compound of Formula I has a particle size of D10 ≤ 3 μm, D50 ≤ 15 μm, D90 ≤ 140 μm, and the compound of Formula I is in amorphous form.

**19.** A pharmaceutical composition, **characterized by** comprising the following components:

| name | mass, parts |
|---|---|
| 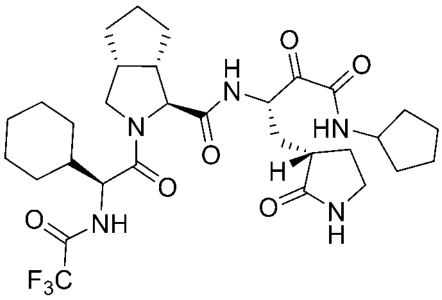 compound of Formula I | 1 |
| lactose | 0.582 |
| microcrystalline cellulose | 1.163 |
| hydroxypropyl methylcellulose | 0.03 |
| colloidal silica | 0.015 |
| cross-linked sodium carboxymethyl cellulose - intragranular | 0.09 |
| magnesium stearate - intragranular | 0.015 |
| cross-linked sodium carboxymethyl cellulose - extragranular | 0.09 |

(continued)

| name | mass, parts |
|---|---|
| magnesium stearate - extragranular | 0.015 |

; the compound of Formula I has a particle size of D10 ≤ 3 μm, D50 ≤ 15 μm, D90 ≤ 140 μm; the compound of Formula I is in amorphous form; and the pharmaceutical composition has a moisture content of 3.0% to 5.0%.

**20.** A pharmaceutical composition, **characterized by** comprising the following components:

| name | mass, parts |
|---|---|
| 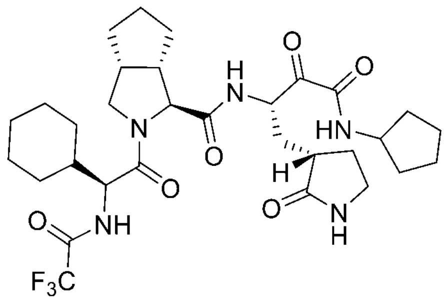 compound of Formula I | 1 |
| lactose | 0.884 |
| microcrystalline cellulose | 0.866 |
| hydroxypropyl methylcellulose | 0.03 |
| colloidal silica | 0.015 |
| cross-linked sodium carboxymethyl cellulose - intragranular | 0.09 |
| magnesium stearate - intragranular | 0.015 |
| cross-linked sodium carboxymethyl cellulose - extragranular | 0.09 |
| magnesium stearate - extragranular | 0.015 |

; the compound of Formula I has a particle size of D10 ≤ 3 μm, D50 ≤ 15 μm, D90 ≤ 140 μm; the compound of Formula I is in amorphous form; and the pharmaceutical composition has a moisture content of 3.0% to 5.0%.

**21.** A pharmaceutical composition, **characterized by** comprising the following components:

| name | mass, parts |
|---|---|
| 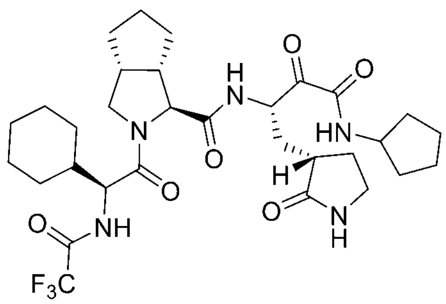 compound of Formula I | 1 |
| lactose | 1.032 |
| microcrystalline cellulose | 0.713 |

(continued)

| name | mass, parts |
|---|---|
| hydroxypropyl methylcellulose | 0.03 |
| colloidal silica | 0.015 |
| cross-linked sodium carboxymethyl cellulose - intragranular | 0.12 |
| magnesium stearate - intragranular | 0.03 |
| cross-linked sodium carboxymethyl cellulose - extragranular | 0.12 |
| magnesium stearate - extragranular | 0.03 |

; the compound of Formula I has a particle size of D10 ≤ 3 μm, D50 ≤ 15 μm, D90 ≤ 140 μm; the compound of Formula I is in amorphous form; and the pharmaceutical composition has a moisture content of 3.0% to 5.0%.

**22.** A pharmaceutical composition, **characterized by** comprising the following components:

| name | mass, parts |
|---|---|
| 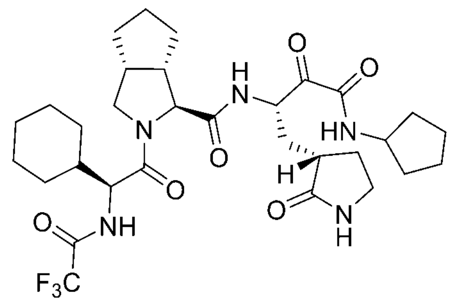 compound of Formula I | 1 |
| lactose | 0.582 |
| microcrystalline cellulose | 1.163 |
| hydroxypropyl methylcellulose | 0.03 |
| colloidal silica | 0.015 |
| cross-linked sodium carboxymethyl cellulose - intragranular | 0.09 |
| magnesium stearate - intragranular | 0.015 |
| cross-linked sodium carboxymethyl cellulose - extragranular | 0.09 |
| magnesium stearate - extragranular | 0.015 |

; the compound of Formula I has a particle size of D10 ≤ 3 μm, D50 ≤ 15 μm, D90 ≤ 140 μm; the compound of Formula I is in amorphous form; and the pharmaceutical composition exhibits a dissolution rate of 70.0% or more at 60 min in a phosphate buffer solution with a pH of 6.8 as a dissolution medium.

**23.** A pharmaceutical composition, **characterized by** comprising the following components:

| name | mass, parts |
|---|---|
| 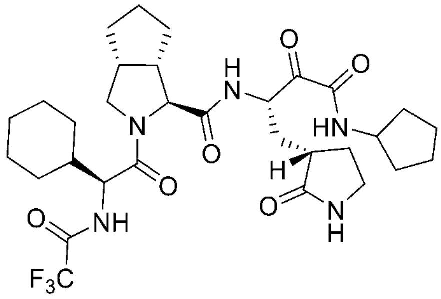 compound of Formula I | 1 |
| lactose | 0.884 |
| microcrystalline cellulose | 0.866 |
| hydroxypropyl methylcellulose | 0.03 |
| colloidal silica | 0.015 |
| cross-linked sodium carboxymethyl cellulose - intragranular | 0.09 |
| magnesium stearate - intragranular | 0.015 |
| cross-linked sodium carboxymethyl cellulose - extragranular | 0.09 |
| magnesium stearate - extragranular | 0.015 |

; the compound of Formula I has a particle size of D10 ≤ 3 μm, D50 ≤ 15 μm, D90 ≤ 140 μm; the compound of Formula I is in amorphous form; and the pharmaceutical composition exhibits a dissolution rate of 70.0% or more at 60 min in a phosphate buffer solution with a pH of 6.8 as a dissolution medium.

**24.** A pharmaceutical composition, **characterized by** comprising the following components:

| name | mass, parts |
|---|---|
| 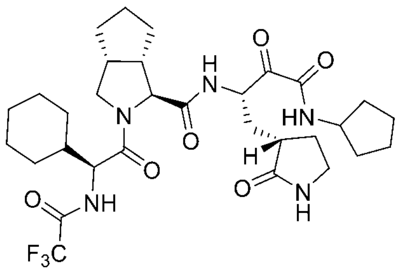 compound of Formula I | 1 |
| lactose | 1.032 |
| microcrystalline cellulose | 0.713 |
| hydroxypropyl methylcellulose | 0.03 |
| colloidal silica | 0.015 |
| cross-linked sodium carboxymethyl cellulose - intragranular | 0.12 |
| magnesium stearate - intragranular | 0.03 |
| cross-linked sodium carboxymethyl cellulose - extragranular | 0.12 |
| magnesium stearate - extragranular | 0.03 |

; the compound of Formula I has a particle size of D10 ≤ 3 μm, D50 ≤ 15 μm, D90 ≤ 140 μm; the compound of Formula I is in amorphous form; and the pharmaceutical composition exhibits a dissolution rate of 70.0% or more at 60 min in a phosphate buffer solution with a pH of 6.8 as a dissolution medium.

**25.** A preparation method for the pharmaceutical composition of any one of claims 1 to 24, **characterized in that** the preparation method is a non-wet granulation preparation technology.

**26.** A pharmaceutical tablet, **characterized by** comprising a core and a coating covering the core, the core is composed of the pharmaceutical composition of any one of claims 1 to 24.

**27.** The pharmaceutical tablet according to claim 26, wherein the coating is a gastric-soluble film coating.

**28.** The pharmaceutical tablet according to claim 27, wherein a mass of the coating is 0.5%-10% of a mass of the core, preferably 1%-6%, more preferably 3%.

**29.** Use of the pharmaceutical composition of any one of claims 1 to 24 or the pharmaceutical tablet of any one of claims 26 to 28 in the preparation of a product for preventing, alleviating or treating an infection or a disease caused by SARS-CoV-2.

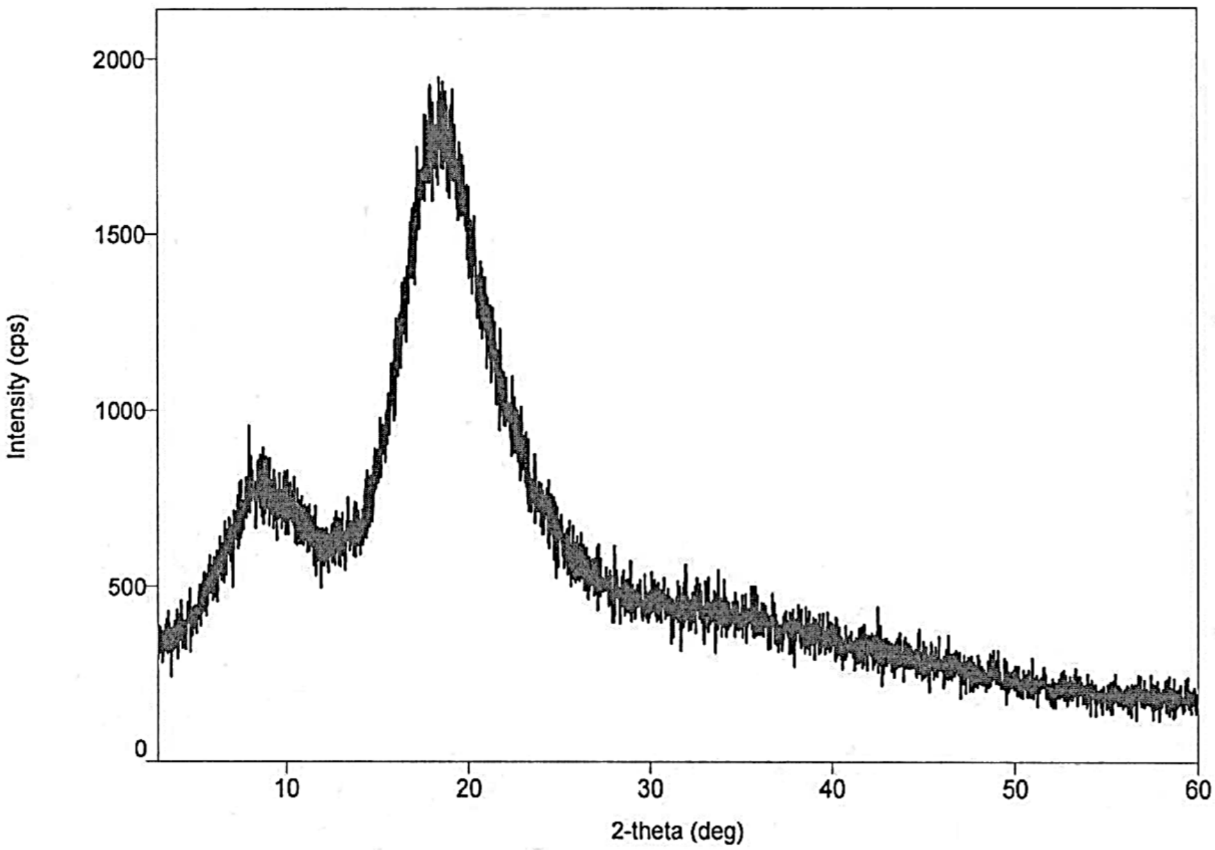
2000
1500
1000
500
0
Intensity (cps)
10
20
30
40
50
60
2-theta (deg)

FIG. 1

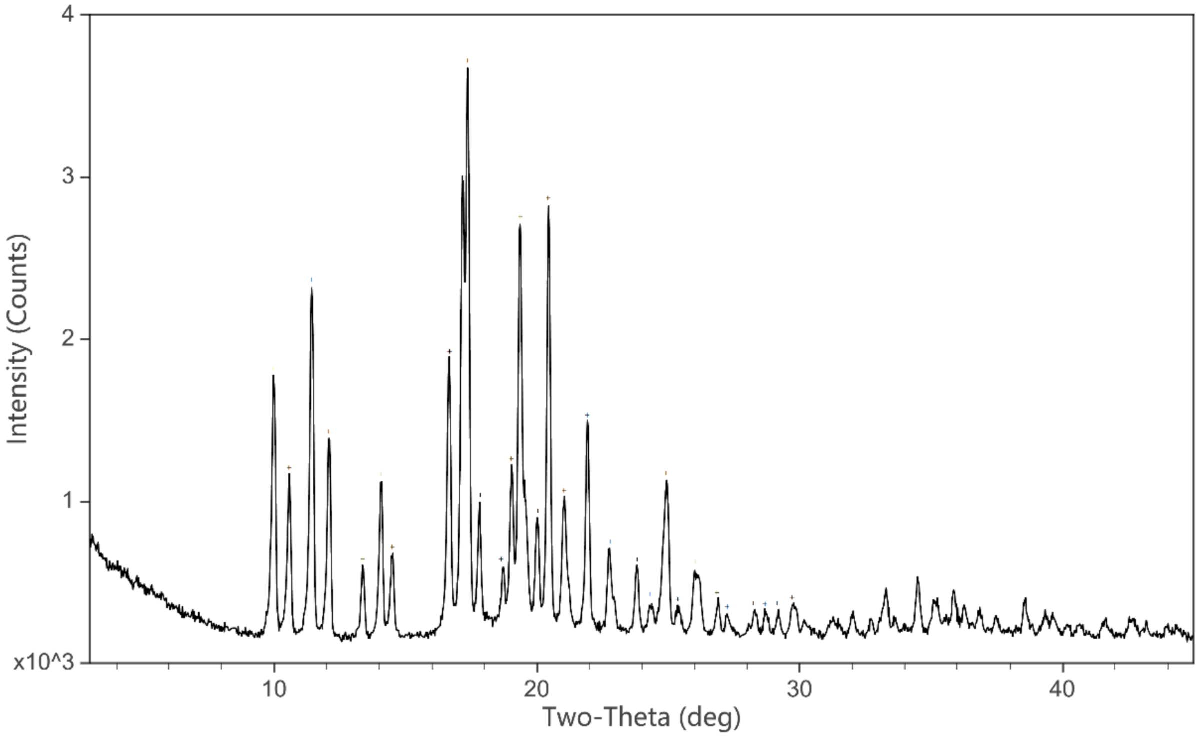
4
3
2
1
x10^3
Intensity (Counts)
10
20
30
40
Two-Theta (deg)

FIG. 2

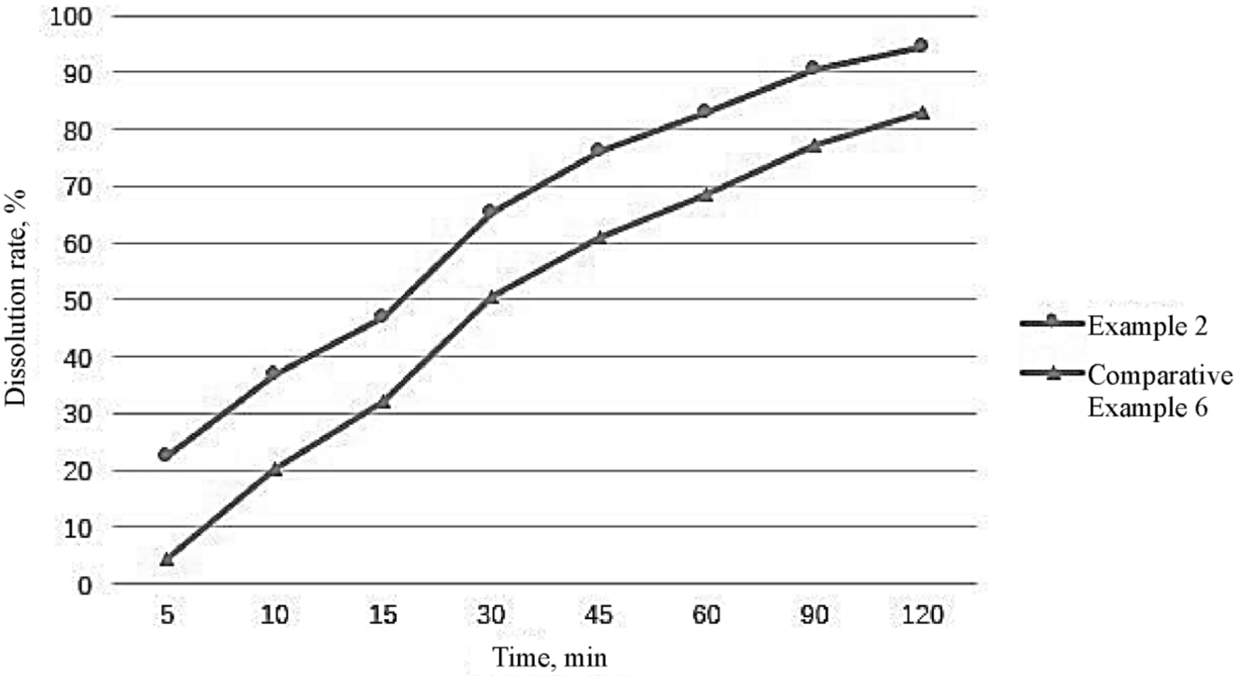

100
90
80
70
60
50
40
30
20
10
0
Dissolution rate, %
5
10
15
30
45
60
90
120
Time, min
Example 2
Comparative Example 6


FIG. 3

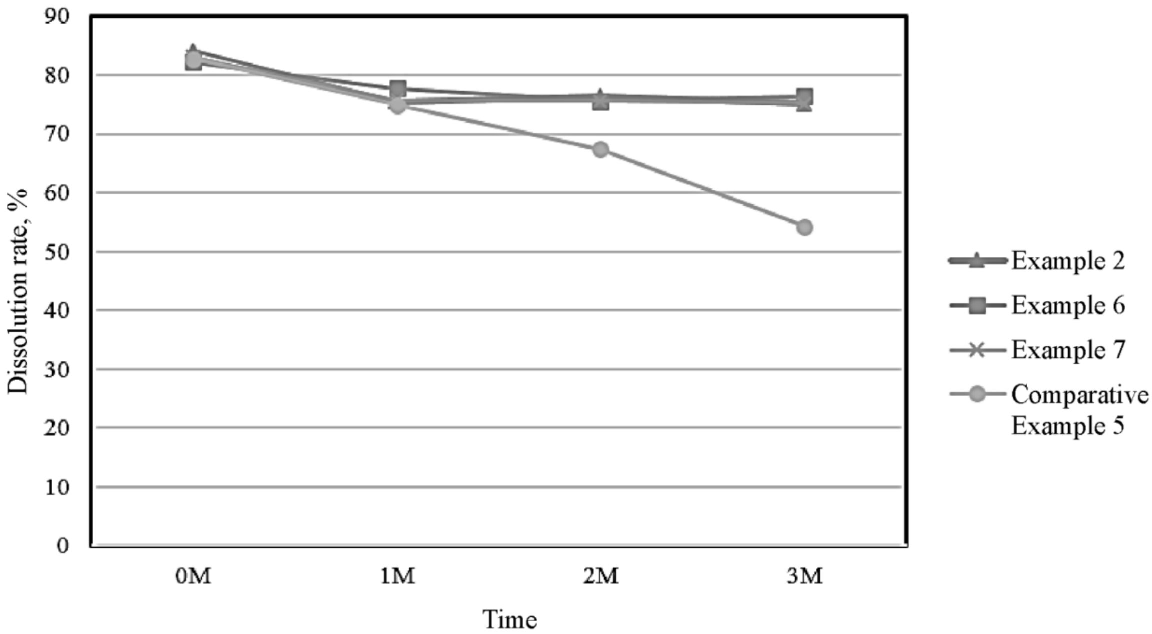

90
80
70
60
50
40
30
20
10
0
Dissolution rate, %
0M
1M
2M
3M
Time
Example 2
Example 6
Example 7
Comparative Example 5


FIG. 4

**INTERNATIONAL SEARCH REPORT**

International application No.
**PCT/CN2024/089353**

**A. CLASSIFICATION OF SUBJECT MATTER**

A61K9/28(2006.01)i; A61K31/403(2006.01)i; A61K31/4015(2006.01)i; A61K31/4025(2006.01)i; A61P31/14(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS, CNTXT, ENTXTC, ENTXT, WOWTXT, USTXT, EPTXT, CNKI, 万方, WANFANG, PUBMED, ISI WEB OF KNOWLEDGE, BING, 结构式式I, 填充剂, 稀释剂, filler, diluent, 乳糖, 微晶纤维素, 甘露醇, lactose, microcrystalline cellulose, 粘合剂, 黏合剂, 羟丙甲纤维素, 羟丙基甲基纤维素, 羟丙基纤维素, 聚维酮, bonding, hydroxypropyl methylcellulose, 助流剂, 助滑剂, 胶态二氧化硅, 胶体二氧化硅, 滑石粉, 微粉硅胶, colloidal silica, glidant, 崩解剂, 低取代羟丙基纤维素, 交联羧甲基纤维素钠, 羧甲基淀粉钠, cross-linked carboxymethyl cellulose sodium, disintegrant, 润滑剂, 硬脂酸镁, 硬脂酸富马酸钠, magnesium stearate, lubricant, 干法, 溶出, 无定型, 新冠, 冠状病毒, SARS-CoV-2, 3C样蛋白酶, 3CLpro, 主要蛋白酶, Mpro, 来瑞特韦, 乐睿灵, leritrelvir, RAY1216, 广东众生睿创生物科技有限公司

**C. DOCUMENTS CONSIDERED TO BE RELEVANT**

| Category* | Citation of document, with indication, where appropriate, of the relevant passages | Relevant to claim No. |
|---|---|---|
| Y | 药智数据 (Non-official translation: Yaozhi). "来瑞特韦片说明书 (Non-official translation: Instructions of Leritrelvir Tablets)" *db.yaozh.com/instruct?bianma=faiRaWNjbGU*, 21 March 2023 (2023-03-21), pages 1-10 | 1-15, 25-29 |
| Y | CN 114668737 A (LEPU PHARMACEUTICALS TECHNOLOGY CO., LTD.) 28 June 2022 (2022-06-28) description, paragraphs 22-48, and embodiments 4-5 | 1-15, 25-29 |
| Y | CN 115594734 A (GUANGDONG RAYNOVENT BIOTECH CO., LTD.) 13 January 2023 (2023-01-13) description, paragraph 59, paragraphs 62-66, paragraph 76, embodiment 1, and experimental examples 1-2 | 1-15, 25-29 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 July 2024** | **24 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/089353**

**C. DOCUMENTS CONSIDERED TO BE RELEVANT**

| Category* | Citation of document, with indication, where appropriate, of the relevant passages | Relevant to claim No. |
|---|---|---|
| Y | CHEN, Xiaoxin et al. "Inhibition mechanism and antiviral activity of an α-ketoamide based SARS-CoV-2 main protease inhibitor" *BioRxiv*, 09 March 2023 (2023-03-09), abstract, and pages 8-11 | 1-15, 25-29 |
| Y | CN 115192536 A (JIANGSU AOSAIKANG PHARMACEUTICAL CO., LTD. et al.) 18 October 2022 (2022-10-18) claims 1-9, and description, paragraphs 6-39 | 1-15, 25-29 |
| Y | CN 114466838 A (PFIZER INC.) 10 May 2022 (2022-05-10) description, paragraph 6, paragraphs 367-368, paragraph 515, and embodiment 13 | 1-15, 25-29 |
| Y | CN 115429770 A (ANHUI BIOCHEM BIO-PHARMACEUTICAL CO., LTD.) 06 December 2022 (2022-12-06) claims 1-10, and description, paragraphs 5-42 | 1-15, 25-29 |
| Y | WO 2022166778 A1 (JIANGSU HENGRUI MEDICINE CO., LTD. et al.) 11 August 2022 (2022-08-11) description, page 2, paragraph 3-page 7, paragraph 1 | 1-15, 25-29 |
| Y | CN 115381786 A (SHANGHAI YINGLI PHARMACEUTICAL CO., LTD.) 25 November 2022 (2022-11-25) description, paragraphs 9-171 | 1-15, 25-29 |
| A | VEGIVINTI, C. T. R et al. "Efficacy of antiviral therapies for COVID-19: a systematic review of randomized controlled trials" *BMC Infectious Diseases*, Vol. 22, 31 January 2022 (2022-01-31), pages 1-45 | 1-29 |

Form PCT/ISA/210 (second sheet) (July 2022)

**INTERNATIONAL SEARCH REPORT**
**Information on patent family members**

International application No.
**PCT/CN2024/089353**

| Patent document cited in search report | Publication date (day/month/year) | Patent family member(s) | Publication date (day/month/year) |
|---|---|---|---|
| CN 114668737 A | 28 June 2022 | None | |
| CN 115594734 A | 13 January 2023 | TW 202311246 A | 16 March 2023 |
| | | TWI 827245 B | 21 December 2023 |
| | | KR 20240029066 A | 05 March 2024 |
| | | WO 2023036093 A1 | 16 March 2023 |
| | | EP 4357342 A1 | 24 April 2024 |
| | | IL 310117 A | 01 March 2024 |
| CN 115192536 A | 18 October 2022 | None | |
| CN 114466838 A | 10 May 2022 | IL 287880 A | 01 January 2022 |
| | | IL 287880 B1 | 01 April 2024 |
| | | MY 196455 A | 12 April 2023 |
| | | KR 20220045251 A | 12 April 2022 |
| | | CO 2021015067 A2 | 17 January 2022 |
| | | DOP 2023000168 A | 15 October 2023 |
| | | US 2022142976 A1 | 12 May 2022 |
| | | US 11541034 B2 | 03 January 2023 |
| | | ECSP 21080528 A | 31 January 2022 |
| | | EP 3953330 A1 | 16 February 2022 |
| | | EP 3953330 B1 | 12 October 2022 |
| | | TW 202317540 A | 01 May 2023 |
| | | US 2022257563 A1 | 18 August 2022 |
| | | US 11452711 B2 | 27 September 2022 |
| | | JP 2022166188 A | 01 November 2022 |
| | | CL 2021002965 A1 | 28 January 2022 |
| | | HRP 20221379 T1 | 06 January 2023 |
| | | AU 2022221493 A1 | 13 October 2022 |
| | | AU 2022221493 B2 | 28 March 2024 |
| | | MD 3953330 T2 | 31 January 2023 |
| | | HUE 060811 T2 | 28 April 2023 |
| | | AU 2021266232 B1 | 03 February 2022 |
| | | AU 2021266232 C1 | 23 June 2022 |
| | | GEP 20247607 B | 11 March 2024 |
| | | WO 2021250648 A1 | 16 December 2021 |
| | | JP 2022534186 A | 28 July 2022 |
| | | JP 7126628 B2 | 26 August 2022 |
| | | AU 2022202158 A1 | 21 April 2022 |
| | | AU 2022202158 B2 | 02 June 2022 |
| | | RS 63714 B1 | 30 November 2022 |
| | | CR 20210558 A | 24 January 2022 |
| | | PT 3953330 T | 06 December 2022 |
| | | US 2022062232 A1 | 03 March 2022 |
| | | US 11351149 B2 | 07 June 2022 |
| | | ZA 202204779 B | 26 October 2022 |
| | | BR 122023004755 B1 | 30 January 2024 |
| | | BR 112021022419 A2 | 10 May 2022 |
| | | BR 112021022419 B1 | 25 April 2023 |
| | | ZA 202204781 B | 26 October 2022 |
| | | CA 3137824 A1 | 03 March 2022 |
| | | CA 3137824 C | 08 November 2022 |
| | | ES 2932173 T3 | 16 January 2023 |

Form PCT/ISA/210 (patent family annex) (July 2022)

**INTERNATIONAL SEARCH REPORT**
**Information on patent family members**

International application No.
**PCT/CN2024/089353**

| Patent document cited in search report | | | Publication date (day/month/year) | Patent family member(s) | | | Publication date (day/month/year) |
|---|---|---|---|---|---|---|---|
| | | | | US | 2023120254 | A1 | 20 April 2023 |
| | | | | ZA | 202108834 | B | 26 April 2023 |
| | | | | NZ | 782196 | A | 26 August 2022 |
| | | | | LT | 3953330 | T | 12 December 2022 |
| | | | | GEP | 20237515 | B | 12 June 2023 |
| | | | | TW | 202214604 | A | 16 April 2022 |
| | | | | TWI | 790704 | B | 21 January 2023 |
| | | | | EP | 4166539 | A1 | 19 April 2023 |
| | | | | MX | 2021013679 | A | 24 January 2022 |
| | | | | UY | 39372 | A | 31 March 2022 |
| | | | | PE | 20220432 | A1 | 29 March 2022 |
| | | | | IL | 299071 | A | 01 February 2023 |
| | | | | PL | 3953330 | T3 | 16 January 2023 |
| | | | | DK | 3953330 | T3 | 24 October 2022 |
| | | | | SI | 3953330 | T1 | 31 January 2023 |
| | | | | KR | 20220031547 | A | 11 March 2022 |
| | | | | KR | 102481876 | B1 | 29 December 2022 |
| CN | 115429770 | A | 06 December 2022 | None | | | |
| WO | 2022166778 | A1 | 11 August 2022 | TW | 202245772 | A | 01 December 2022 |
| CN | 115381786 | A | 25 November 2022 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN ZL202211095326
- CN 2023120437 W
- CN 2023120726 W